# EUROPEAN PATENT SPECIFICATION

(11) **EP 3 152 577 B1**
(45) Date of publication and mention of the grant of the patent: **18.07.2018**
(21) Application number: 15726957.2
(22) Date of filing: 03.06.2015
(51) Int. Cl.: C12Q 1/6886, G01N 33/574

(54) **SIGNIFICANCE OF INTRATUMORAL HER2 HETEROGENEITY IN BREAST CANCER AND USES THEREFOR**
BEDEUTUNG DER INTRATUMORALEN HER2 HETEROGENITÄT IN BRUSTKREBS UND DEREN VERWENDUNG
IMPORTANCE DE INTRATUMORALE HER2 HÉTÉROGÉNÉITÉ EN CANCER DU SEIN ET LEUR UTILISATIONS

(30) Priority: 06.06.2014 US 201462009057 P
(43) Date of publication of application: 12.04.2017
(73) Proprietor: Ventana Medical Systems, Inc., Tucson, AZ 85755 (US)
(72) Inventor: NITTA, Hiro, Tucson, AZ 85718 (US); PADILLA, Mary T., Livermore, CA 94550 (US); RANGER-MOORE, James, Tucson, AZ 85719 (US); DENNIS, Eslie, Tucson, AZ 85704 (US); KUROZUMI, Sasagu, 330-0063 Saitama (JP); KUROSUMI, Masafumi, 330-0063 Saitama (JP)
(74) Representative: Burger, Alexander
(86) International application number: PCT/EP2015/062331
(87) International publication number: WO 2015/185595

(56) References cited:
- WO-A1-2012/024185
- WO-A2-2008/043104
- US-A1- 2012 052 508
- US-A1- 2013 260 379
- HIROAKI NITTA ET AL: "A gene-protein assay for human epidermal growth factor receptor 2 (HER2): brightfield tricolor visualization of HER2 protein, the HER2 gene, and chromosome 17 centromere (CEN17) in formalin-fixed, paraffin-embedded breast cancer tissue sections", DIAGNOSTIC PATHOLOGY, BIOMED CENTRAL LTD, LO, vol. 7, no. 1, 30 May 2012 (2012-05-30), page 60, XP021122331, ISSN: 1746-1596, DOI: 10.1186/1746-1596-7-60
- Y NISHIDA ET AL: "Intratumoral Heterogeneity in Gastric Cancer Revealed by Simultaneous Analysis of Protein Over-Expression and Gene Amplification Using a Gene-Protein Assay Modern Pathology", MODERN PATHOLOGY, vol. 27, no. Suppl 2, 1 February 2014 (2014-02-01), page 195A, XP055206366,
- HEE EUN LEE ET AL: "Clinical significance of intratumoral HER2 heterogeneity in gastric cancer", EUROPEAN JOURNAL OF CANCER, vol. 49, no. 6, 1 April 2013 (2013-04-01), pages 1448-1457, XP055206417, ISSN: 0959-8049, DOI: 10.1016/j.ejca.2012.10.018
- HOFMANN M ET AL: "Assessment of a HER2 scoring system for gastric cancer: results from a validation study", HISTOPATHOLOGY, WILEY-BLACKWELL PUBLISHING LTD, GB, vol. 52, no. 7, 1 June 2008 (2008-06-01), pages 797-805, XP002600918, ISSN: 0309-0167, DOI: 10.1111/J.1365-2559.2008.03028.X
- MONTEMURRO FILIPPO ET AL: "Potential biomarkers of long-term benefit from single-agent trastuzumab or lapatinib in HER2-positive metastatic breast cancer", MOLECULAR ONCOLOGY, ELSEVIER, AMSTERDAM, NL, vol. 8, no. 1, 13 September 2013 (2013-09-13), pages 20-26, XP028816117, ISSN: 1574-7891, DOI: 10.1016/J.MOLONC.2013.08.013
- NITTA HIROAKI ET AL: "Development of automated brightfield double In Situ hybridization (BDISH) application for HER2 gene and chromosome 17 centromere (CEN 17) for breast carcinomas and an assay performance comparison to manual dual color HER2 fluorescence In Situ hybridization (FISH)", DIAGNOSTIC PATHOLOGY, BIOMED CENTRAL LTD, LO, vol. 3, no. 1, 22 October 2008 (2008-10-22), page 41, XP021045226, ISSN: 1746-1596, DOI: 10.1186/1746-1596-3-41
- Gail H Vance ET AL: "Genetic heterogeneity in HER2 testing in breast cancer: panel summary and guidelines", Archives of pathology & laboratory medicine, 1 April 2009 (2009-04-01), page 611, XP055206427, United States Retrieved from the Internet: URL:http://www.ncbi.nlm.nih.gov/pubmed/193 91661 [retrieved on 2015-08-04]

## Description

### FIELD

This disclosure relates to methods of measuring tissue heterogeneity and using the same as a prognostic and predictive tool in the diagnosis and treatment of breast cancer.

### BACKGROUND

Breast cancer accounts for about 23% of all cancers worldwide, and is responsible for hundreds of thousands of deaths each year. Breast cancers vary in their response to different treatments and it is important to select an appropriate treatment regimen for each patient. Receptor status is a common classification system that is used to select treatments for a patient with breast cancer. Breast tumors may have (be positive for) or lack (be negative for) estrogen receptor (ER) protein, HER2 (also known as ErbB2) protein, and/or progesterone receptor (PR) protein. Breast tumors are also routinely screened for HER2 gene amplification, as another measure of whether the tumor is HER2 positive or negative. Some breast tumors are negative for all three markers and are referred to as "triple negative" tumors.

Estrogen receptor (ER) and/or progesterone receptor (PR) positive tumors are typically treated with hormone-blocking therapy (such as tamoxifen), while HER2 positive tumors are treated with HER2-targeting therapeutics such as trastuzumab or lapatinib. Although current methods of breast cancer classification and targeted treatment have improved patient outcomes, many HER2 positive tumors do not respond to, or acquire resistance to, HER2-targeting therapies. Current HER2 screening methods may produce false positive results, due in part to tumor heterogeneity. Thus, there remains a need to improve current molecular screening methods to rapidly and accurately classify breast tumors and to select appropriate therapies in the clinic.

Scoring HER2, either HER2 gene amplification or HER2 protein overexpression alone, has been used as a guide for HER2-targeted therapies. Nitta et al. (Diagn Pathol. 7:60, 2012) describe a gene-protein assay for human epidermal growth factor receptor 2 (HER2) in formalin-fixed, paraffin-embedded breast cancer tissue sections. Nishida et al. (Mod Pathol. 27 (suppl 2):195A, 2014) investigate intratumoral heterogeneity in gastric cancer by simultaneous analysis of protein over-expression and gene amplification using a gene-protein assay. Lee et al. (Eur J of Cancer. 49, 1448, 2013) evaluate the clinical significance of intratumoral HER2 heterogeneity in gastric cancer. Hofmann et al. (Histopathol. 52:797, 2008) assess a HER2 scoring system for gastric cancer. While these assays have been very beneficial to breast cancer patients, new assays capable of further stratifying or predicting a patient's response to a therapy are continually being sought.

### SUMMARY

In illustrative embodiments, a method for predicting responsiveness to a HER2-directed therapy by assessing HER2 heterogeneity in a tumor is provided, the method comprising: contacting a sample of the tumor with an antibody that specifically binds to HER2 protein and detecting HER2 protein in the sample, contacting the sample of the tumor with a nucleic acid probe that specifically binds HER2 genomic DNA and detecting HER2 gene amplification status in the sample, and scoring the HER2 protein (IHC) and HER2 gene (DISH). The scoring is categorized as: Group A for samples exhibiting IHC 3+ and DISH+, Group B for samples exhibiting IHC 3+ and DISH-, Group C for samples exhibiting IHC 2+ and DISH+, Group D for samples exhibiting IHC 2+ and DISH-, Group E for samples exhibiting IHC 0, 1+ and DISH+, and Group F for samples exhibiting IHC 0, 1+ and DISH-. The method further comprises predicting that the tumor is responsive to the HER2-directed therapy if the tumor reveals a first foci having a first score being Group F and a second foci having a second score selected from Group A to Group E.

In another embodiment, the method further comprises assaying a second sample of the tumor for estrogen receptor (ER) and progesterone receptor (PR), wherein the step of predicting that the tumor is responsive to the HER2-directed therapy comprises predicting that the tumor is responsive to the HER2-directed therapy if the ER and PR are negative so that the tumor is understood to be triple negative breast cancer (TNBC). In other words, the tumor is predicted as being responsive to the HER2-directed therapy if the ER and PR are negative so that the tumor is understood to be TNBC. In yet another embodiment, the method further comprises contacting the sample of the tumor with an antibody that specifically binds to estrogen receptor (ER) protein and detecting ER protein in the sample, contacting the sample of the tumor with an antibody that specifically binds to progesterone receptor (PR) protein and detecting PR protein in the sample. The method further includes predicting that the tumor is responsive to the HER2-directed therapy if the ER and PR are negative so that the tumor is understood to be triple negative breast cancer (TNBC). In another embodiment, the HER-2 directed therapy is selected from the group consisting of trastuzumab, trastuzumab emtansine, pertuzumab, neratinib, and lapatinib.

In illustrative embodiments, a method of scoring a tumor sample is provided, the method comprising: contacting the tumor sample with an antibody that specifically binds to HER2 protein and detecting HER2 protein in the sample, contacting the tumor sample with a nucleic acid probe that specifically binds HER2 genomic DNA and detecting HER2 gene amplification status in the sample, scoring the HER2 protein (IHC) and HER2 gene (DISH) according to the aforementioned Groups A-F. The method further comprises scoring the tumor sample as heterogeneous if the tumor reveals a first foci being Group F and a second foci having a second score selected from Group A to Group E.

In another embodiment, the method further comprises prognosing a hazard ratio of greater than 5 if the sample is scored as heterogeneous. In yet another embodiment, the method further comprises assaying a second sample of the tumor for estrogen receptor (ER) and progesterone receptor (PR), wherein the tumor is predicted as being responsive to the HER2-directed therapy if the ER and PR are negative so that the tumor is understood to be triple negative breast cancer (TNBC). In yet another embodiment, the method further comprises contacting the sample of the tumor with an antibody that specifically binds to estrogen receptor (ER) protein and detecting ER protein in the sample; contacting the sample of the tumor with an antibody that specifically binds to progesterone receptor (PR) protein and detecting PR protein in the sample, wherein the tumor is predicted as being responsive to the HER2-directed therapy if the ER and PR are negative so that the tumor is understood to be triple negative breast cancer (TNBC). In one embodiment, the method further comprises prognosing a significantly worse survival score compared to a non-heterogeneous score (RFS: P=0.0176; CSS: P=0.0199) if the sample is scored as heterogeneous.

### BRIEF DESCRIPTION OF THE DRAWINGS

The following figures are submitted in color.
**FIGS. 1A and B** are a pair of images of a breast tumor tissue sample stained for HER2 gene (black dots), HER2 protein (brown color), and ER protein (red color) at 4Xmagnification (FIG. 1A) and 60X magnification (FIG. 1B). The sample is HER2 gene amplified, HER2 protein positive, and ER protein positive. However, some cells (circled) are negative for HER2 protein, though they are ER protein positive and have HER2 gene amplification.
**FIGS. 2A and B** are a pair of images of a breast tumor tissue sample stained for HER2 gene (black dots), HER2 protein (brown color), and ER protein (red color) at 4X magnification (FIG. 2A) and 60X magnification (FIG. 2B). The sample has amplified HER2 gene and is ER protein positive, but is HER2 protein negative, as evidence by the faint or absent brown staining.
**FIGS. 3A and B** are a pair of images of a breast tumor tissue sample stained for HER2 gene (black dots), HER2 protein (brown color), and ER protein (red color) at 4X magnification (FIG. 3A) and 60X magnification (FIG. 3B). The sample shows HER2 gene amplification and is HER2 protein positive, but is ER negative, as evidenced by the lack of red staining. The red staining in FIG. 3B is ER protein staining in normal mammary gland cells in the sample.
**FIGS. 4A-C** are a series of images showing ER protein IHC with iVIEW DAB staining (FIG. 4A) or ULTRAVIEW Red staining (FIG. 4B) and HER2 gene and protein IHC/ISH with ULTRAVIEW Red IHC staining (FIG. 4C) in a breast tissue sample, at 20X magnification.
**FIGS. 5A-C** are a series of images showing Ki67 protein IHC with iVIEW DAB staining (FIG. 5A) or ULTRAVIEW Red staining (FIG. 5B) and HER2 gene and protein IHC/ISH with ULTRAVIEW Red IHC staining (FIG. 5C) in a breast tissue sample, at 20X magnification.
**FIG. 6** is an image of exemplary detection of HER2 gene (black dots), HER2 protein (brown color), and Ki67 (red color) in a breast tissue sample.
**FIGS. 7A-D** are a series of images of staining of HER2 protein (brown staining), HER2 gene (black dots), and Ki67 protein (red staining) (FIGS. 7A and C) or HER2 protein (brown staining), HER2 gene (black dots), and ER protein (red staining) (FIGS. 7B and D) in a breast tissue sample at 20X magnification (FIGS. 7A and B) or 60X magnification (FIGS. 7C and D).
**FIGS. 8A-C** are a series of images showing HER2 gene (black dots), HER2 protein (brown staining), and ER protein (red staining) in a HER2 equivocal breast tissue sample. FIG. 8B shows the sample at 10X magnification. The boxed red area in FIG. 8B is shown in FIG. 8A at 60X magnification and the boxed blue area in FIG. 8B is shown in FIG. 8C at 60X magnification.
**FIGS. 9A-C** are a series of images showing HER2 gene (black dots), HER2 protein (brown staining), and ER protein (red staining) in a HER2 positive breast tissue sample. FIG. 9B shows the sample at 10X magnification. The boxed red area in FIG. 9B is shown in FIG. 9A at 60X magnification and the boxed blue area in FIG. 9B is shown in FIG. 9C at 60X magnification.
**FIGS. 10A and B** are a pair of images showing staining of HER2 protein (brown), ER protein (purple), HER2 gene (black spots), and chromosome 17 centromere DNA (red spots) in an exemplary HER2 positive/ER positive breast tissue sample at 20X magnification (FIG. 10A) and 60X magnification (FIG. 10B).
**FIGS. 11A and B** are a pair of images showing staining of HER2 protein (brown), ER protein (purple), HER2 gene (black spots), and chromosome 17 centromere DNA (red spots) in an exemplary HER2 negative/ER positive breast tissue sample at 20X magnification (FIG. 11A) and at 60X magnification (FIG. 11B).
**FIGS. 12A-C** are three photomicrographs of a cervical dysplasia case in which 12A uses a stringency wash of 68°C, 12B uses a stringency wash of 72°C, and 12C uses a stringency wash of 76°C.
**FIGS. 13A-C** show three photomicrographs of a ZR-75-1 xenograft tumor in which 13A uses a stringency wash of 68°C, 13B uses a stringency wash of 72°C, and 13C uses a stringency wash of 76°C.
**FIGS. 14A-B** are photomicrographs of the HER2 Gene-Protein Assay employing a dual stringency wash approach in which FIG. 14A shows a ZR-75-1 xenograft tumor and FIG. 14B shows a cervical dysplasia case.
**FIGS. 15A-B** show the HER2 Gene-Protein Assay employing a dual stringency wash approach in which FIG. 15A shows a breast cancer tumor at Objective 4X and FIG. 15B shows the same case at Objective 100X.
**FIGS. 16A-B** show the HER2 Gene-Protein Assay employing a dual stringency wash approach in which FIG. 16A shows a breast cancer tumor at Objective 4X and FIG. 16B shows the same case at Objective 100X.
**FIGS. 17A-B** are a graph (FIG. 17A) and table (FIG. 17B) that show regression free survival (RFS) by a clinical trial group as determined by the gene-protein assay.
**FIGS. 18A-B** are a graph (FIG. 18A) and table (FIG. 18B) that show cancer-specific survival (CSS) by the clinical trial group as determined by the gene-protein assay.
**FIGS. 19A-B,** are graphs for <RFS> (FIG. 19A) and <CSS> (FIG. 19B) which show the impact of heterogeneity within the context of the gene protein assay on the clinical trial group.
**FIG. 20** shows a sub-population of the data shown in FIG. 19, wherein the population was triple negative breast cancer (TNBC - for ER, PR, and within Group F for gene protein assay).
**FIG. 21A-B,** are photomicrographs of a representative tissue stained according to the gene-protein assay at 10x objective (FIG. 21A) and at 60x (FIG. 21B), which provides evidence as to a biological cause of cancer tumor heterogeneity.

### DETAILED DESCRIPTION

Standard breast tumor classification includes determining tumor status for ER, PR, and HER2 and selection of therapy based on whether the tumor is ER positive, HER2 positive, or is triple negative. However, it has been recognized more recently that a subset of HER2 positive tumors are ER positive, and that such tumors may respond favorably to a combination of anti-estrogen and anti-HER2 therapies (e.g., Rimawi et al., J. Clin. Oncol. 14:1726-1731, 2013; Montemurro et al., Ann. Oncol. doi: 10.1093/annonc/mdt287, 2013; Vaz-Luis et al., Ann. Oncol. 24:283-291, 2013). Thus, accurate identification of HER2 positive/ER positive tumors is becoming increasingly important. In addition, there is increasing recognition of discordance between HER2 protein expression and HER2 gene amplification results and the potential role of tumor heterogeneity in such discordance (e.g., Nitta et al., Diagn. Pathol. 7:60, 2012). Thus, there remains a need for improved assays for accurately identifying HER2 positive tumors, as well as HER2 positive/ER positive tumors.

Tissue heterogeneity (e.g., tumor heterogeneity) confounds cancer diagnoses. In a heterogeneous tissue sample, compiling the results from individual analyses of multiple single markers is inferior to a multiplexed approach on a single sample for several reasons. First, multiplexing makes it possible to identify those cells within the sample that express multiple markers in a population of cells that differentially expresses those single markers heterogeneously. For example, two single marker assays for a sample that heterogeneously expresses markers A and B across the population of cells would establish that, for both markers, there are cells positive and negative for both markers. The two single marker assays will not provide the extent to which the positivity and negativity overlaps within the cells. As such, the extent to which the cells are heterogeneous cannot be known. Using the single marker assays, the extent to which cells are negative for both markers, positive for a single marker, or positive for both markers would not be quantifiable. While this benefit is realized in a dual assay format, the benefits are compounded for higher levels of multiplexing. Even in homogeneous tissues, where multiplexing would not provide such a distinct advantage, multiplexing has other advantages, such as the preservation of sample.

### I. Terms

Suitable methods and materials for the practice and/or testing of embodiments of the disclosure are described below. For example, conventional methods well known in the art to which the disclosure pertains are described in various general and more specific references, including, for example, Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999.

In order to facilitate review of the various embodiments of the disclosure, the following explanations of specific terms are provided:
Antibody: A polypeptide that includes at least a light chain or heavy chain immunoglobulin variable region and specifically binds an epitope of an antigen (such as HER2 protein or ER protein). Antibodies include monoclonal antibodies, polyclonal antibodies, or fragments of antibodies. An antibody can be conjugated or otherwise labeled with a detectable label, such as an enzyme, hapten, or fluorophore.
Detectable label: A molecule or material that can produce a signal (such as a visual, electrical, or other signal) that indicates the presence and/or amount of a target (such as a protein or nucleic acid) in a sample. When conjugated to a specific binding molecule (for example, an antibody or nucleic acid probe), the detectable label can be used to locate and/or quantify the target to which the specific binding molecule is directed. A detectable label can be detected directly or indirectly, and several different detectable labels can be used in combination to detect one or more targets. For example, a first detectable label, such as a hapten conjugated to an antibody specific to a target, can be detected indirectly by using a second detectable label that is conjugated to a molecule that specifically binds the first detectable label. In addition, multiple detectable labels that can be separately detected can be conjugated to different specific binding molecules that specifically bind different targets to provide a multiplex assay that can provide detection of the multiple targets in a single sample.

Detectable labels include chromogenic, fluorescent, phosphorescent and/or luminescent molecules, catalysts (such as enzymes) that convert one substance into another substance to provide a detectable signal (such as by converting a colorless substance into a colored substance or vice versa, or by producing a precipitate or increasing sample turbidity), haptens that can be detected through antibody-hapten binding interactions using additional detectably labeled antibody conjugates, and paramagnetic and magnetic molecules or materials. Particular examples of detectable labels include: enzymes, such as horseradish peroxidase, alkaline phosphatase, acid phosphatase, glucose oxidase, β-galactosidase or β-glucuronidase; fluorophores, such as fluoresceins, luminophores, coumarins, BODIPY dyes, resorufins, and rhodamines (many additional examples of fluorescent molecules can be found in The Handbook - A Guide to Fluorescent Probes and Labeling Technologies, Molecular Probes, Eugene, OR); nanoparticles, such as quantum dots (U.S. Patent Nos. 6,815,064, 6,682,596 and 6,649,138); metal chelates, such as DOTA and DPTA chelates of radioactive or paramagnetic metal ions like Gd3+; and liposomes, for example, liposomes containing trapped fluorescent molecules. Where the detectable label includes an enzyme, a detectable substrate such as a chromogen, a fluorogenic compound, or a luminogenic compound is used in combination with the enzyme to generate a detectable signal (a wide variety of such compounds are commercially available, for example, from Life Technologies, Carlsbad, CA)

Alternatively, an enzyme can be used in a metallographic detection scheme. In some examples, metallographic detection methods include using an enzyme, such as alkaline phosphatase, in combination with a water-soluble metal ion and a redox- inactive substrate of the enzyme. The substrate is converted to a redox-active agent by the enzyme, and the redox-active agent reduces the metal ion, causing it to form a detectable precipitate (see, for example, U.S. Pat. Nos. 7,642,064; 7,632,652). In other examples, metallographic detection methods include using an oxido-reductase enzyme (such as horseradish peroxidase) along with a water soluble metal ion, an oxidizing agent and a reducing agent, again to form a detectable precipitate (see, for example, U.S. Patent No. 6,670,113). Haptens are small molecules that can be bound by antibodies. Exemplary haptens include dinitrophenyl (DNP), biotin, digoxigenin (DIG), and fluorescein. Additional haptens include oxazole, pyrazole, thiazole, nitroaryl, benzofuran, triperpene, urea, thiourea, rotenoid, coumarin and cyclolignan haptens, such as those disclosed in U.S. Pat. No. 7,695,929.

Estrogen receptor (ER): Also known as estrogen receptor 1 (ESR1), estrogen receptor alpha (ER-alpha) estrogen nuclear receptor alpha; GenBank Gene ID Accession No. 2099. A hormone-activated transcription factor. Upon binding to estrogen (or other ER agonists) the estrogen receptor localizes to the nucleus and forms homodimers or heterodimers with estrogen receptor 2 and activates transcription of various genes.

ER nucleic acid and protein sequences are publicly available. For example, the ER gene is located on chromosome 6q25.1 and its sequence is disclosed as GenBank Accession No. NC_000006.11 (152011631-152424409). GenBank Accession Nos. NM_001122742, NM_001122741, NM_001122740, NM_000125, XM_005266856, and XM_005266857 disclose ER nucleic acid sequences, and GenBank Accession Nos.: NP_001116214, NP_001116213, NP_001116212, NP_000116, XP_005266913, and XP_005266914 disclose ER protein sequences, as provided by GenBank on October 4, 2013.

HER2: Also known as v-erb-b2 avian erythroblastic leukemia viral oncogene homolog 2 (ErbB2), human epidermal growth factor receptor 2, Her2/neu, c-erb B2/neu, and neuroblastoma/glioblastoma derived oncogene homolog; GenBank Gene ID Accession No. 2064. As a member of the epidermal growth factor receptor tyrosine kinase family, Her2 heterodimerizes with other ligand-bound EGF receptor family members, though it lacks a ligand binding domain and cannot bind ligands itself. Amplification and/or overexpression of Her2 occur in several types of cancer, including breast and ovarian cancer.

Her2 nucleic acid and protein sequences are publicly available. For example, the Her2 gene is located on chromosome 17q12 and its sequence is disclosed as GenBank Accession No. NC_000017.10 (37844167-37884915). GenBank Accession Nos. NM_001005862, NM_004448, XM_005257139, and XM_005257140 disclose Her2 nucleic acid sequences, and GenBank Accession Nos.: NP_001005862, NP_004439, XP_005257196, and XP_005257197 disclose Her2 protein sequences, as provided by GenBank on October 4, 2013.

Immunohistochemistry (IHC): A method of determining the presence or distribution of an antigen in a sample by detecting interaction of the antigen with a specific binding agent, such as an antibody. A sample is contacted with an antibody detected by means of a detectable label conjugated to the antibody (direct detection) or by means of a detectable label conjugated to a secondary antibody, which binds specifically to the primary antibody (e.g., indirect detection).

Scoring the HER2 protein (IHC): Scoring a sample for HER2 protein using the following FDA criteria for immunohistochemistry (IHC): score 0 (IHC 0), score 1+ (IHC 1), score 2+ (IHC 2+), score 3+ (IHC 3+).

In situ hybridization (ISH): A method of determining the presence or distribution of a nucleic acid in a sample using hybridization of a labeled nucleic acid probe to localize a specific DNA or RNA sequence in a portion or section of tissue (in situ), or, if the tissue is small enough (e.g., plant seeds, Drosophila embryos), in the entire tissue (whole mount ISH). DNA ISH can be used to determine the structure of chromosomes, such as for use in medical diagnostics to assess chromosomal integrity and/or to determine gene copy number in a sample. RNA ISH measures and localizes mRNAs and other transcripts within tissue sections or whole mounts.

For ISH, sample cells and tissues are usually treated to fix the target nucleic acids in place and to increase access of the probe to the target molecule. The detectably labeled probe hybridizes to the target sequence at elevated temperature, and then the excess probe is washed away. Solution parameters, such as temperature, salt and/or detergent concentration, can be manipulated to remove any non-identical interactions (e.g., so only exact sequence matches will remain bound). Then, the labeled probe is localized and potentially quantitated in the tissue using either autoradiography, fluorescence microscopy or immunohistochemistry, respectively. ISH can also use two or more probes, which are typically differently labeled to simultaneously detect two or more nucleic acids.

Dual in situ hybridization (DISH): An in situ hybridization (ISH) method using two probes to detect two different target sequences. Typically, these two probes are differently labeled. In the methods presented herein, DISH may be an assay to determine the HER2 gene amplification status by contacting a sample of a tumor with a HER2-specific probe and a chromosome 17 centromere probe and determining a ratio of HER2 genomic DNA to chromosome 17 centromere DNA (such as a ratio of HER2 gene copy number to chromosome 17 centromere copy number). The method includes utilizing different detectable labels and/or detection systems for each of the HER2 genomic DNA and chromosome 17 centromere DNA, such that each can be individually visually detected in a single sample.

Scoring the HER2 gene (DISH): Scoring a sample for HER2 gene using the following FDA criteria based on the ratio of HER2 genomic DNA to chromosome 17 centromere DNA as determined in a DISH assay: DISH- (negative: HER2/CEN17 < 2) DISH + (positive: HER2/CEN17 ≥ 2.0).

Probe: An isolated nucleic acid (such as an isolated synthetic oligonucleotide), attached to a detectable label or reporter molecule. Typical labels include radioactive isotopes, enzyme substrates, co-factors, ligands, chemiluminescent or fluorescent agents, haptens (including, but not limited to, DNP), and enzymes. Methods for labeling and guidance in the choice of labels appropriate for various purposes are discussed, e.g., in Sambrook et al. (In Molecular Cloning: A Laboratory Manual, CSHL, New York, 1989) and Ausubel et al. (In Current Protocols in Molecular Biology, Greene Publ. Assoc. and Wiley-Intersciences, 1992).

Probes can be selected to provide a desired specificity, and may comprise at least 15, 20, 25, 30, 35, 40, 45, 50 or more nucleotides of a target nucleic acid. In particular examples, probes can include at least 100, 250, 500, 600, 1000, or more nucleotides of a target nucleic acid. In some examples, the probe includes segments of nucleotides that are from non-contiguous portions of a target nucleic acid, such as a HER2 genomic nucleic acid.

Sample: The term "sample" refers to any liquid, semi-solid or solid substance (or material) in or on which a target can be present. In particular, a sample can be a biological sample or a sample obtained from a biological material. Exemplary biological samples include tissue samples and/or cytology samples, for example, obtained from an animal subject, such as a human subject. In other examples, a biological sample can be a biological fluid obtained from, for example, blood, plasma, serum, urine, bile, ascites, saliva, cerebrospinal fluid, aqueous or vitreous humor, or any bodily secretion, a transudate, an exudate (for example, fluid obtained from an abscess or any other site of infection or inflammation), or fluid obtained from a joint (for example, a normal joint or a joint affected by disease). A biological sample can also be a sample obtained from any organ or tissue (including a biopsy or autopsy specimen, such as a tumor biopsy) or can include a cell (whether a primary cell or cultured cell) or medium conditioned by any cell, tissue or organ.

Specific binding: A term that refers to the binding of an agent that preferentially binds to a defined target (such as an antibody to a specific protein or antigen or a nucleic acid probe to a specific nucleic acid sequence). With respect to a target protein, "specifically binds" refers to the preferential association of an antibody or other ligand, in whole or part, with a specific polypeptide. "Specifically binds" refers to the preferential association of a nucleic acid probe, in whole or part, with a specific nucleic acid, when referring to a target nucleic acid.

A specific binding agent binds substantially only to a particular target. A minor amount of non-specific interaction may occur between a specific binding agent and a non-target protein or nucleic acid. Antibody to antigen specific binding typically results in greater than 2-fold, such as greater than 5-fold, greater than 10-fold, or greater than 100-fold increase in amount of bound antibody or other ligand (per unit time) to a target protein, as compared to a non-target protein. Immunoassay formats can be used to select antibodies that specifically react with a particular protein (such as antibodies that specifically bind HER2 protein or ER protein). See Harlow & Lane, Antibodies, A Laboratory Manual, Cold Spring Harbor Publications, New York (1988), for a description of immunoassay formats and conditions.

Specific binding of a nucleic acid probe to a target nucleic acid molecule typically results in greater than 2-fold, such as greater than 5-fold, greater than 10-fold, or greater than 100-fold increase in amount of bound nucleic acid probe to a target nucleic acid as compared to a non-target nucleic acid. A variety of ISH conditions are appropriate for selecting nucleic acid probes that bind specifically with a particular nucleic acid sequence (such as a HER2-specific probe or a chromosome 17 centromere probe).

Subject: Any multi-cellular vertebrate organism, such as human or non-human mammals (e.g., veterinary subjects).

### II. Overview of Several Embodiments

Disclosed herein are methods for detecting multiple target molecules (such as two or more proteins and/or nucleic acids) in a single sample. In particular embodiments, the methods include detecting presence and/or amount of HER2 protein, ER protein, and HER2 genomic DNA (such as HER2 gene copy number) in a single sample. In some embodiments, the methods further include detecting presence and/or amount of chromosome 17 centromere DNA in the sample, and in some examples, determining a ratio of HER2 genomic DNA to chromosome 17 centromere DNA (such as a ratio of HER2 gene copy number to chromosome 17 centromere copy number). The methods include utilizing different detectable labels and/or detection systems for each of the HER2 protein, ER protein, HER2 genomic DNA, and chromosome 17 centromere DNA (if included), such that each can be individually visually detected in a single sample.

In some embodiments of the methods, a sample is contacted with an antibody that specifically binds to HER2 protein and HER2 protein is detected, the sample is contacted with an antibody that specifically binds to ER protein and ER protein is detected, and the sample is contacted with a nucleic acid probe that specifically binds to HER2 genomic DNA and HER2 genomic DNA is detected. The detection of HER2 protein, ER protein, and HER2 genomic DNA can be performed concomitantly or sequentially. In one specific embodiment, the method includes sequentially detecting HER2 protein (contacting the sample with a HER2-specific antibody and detecting HER2 protein in the sample), followed by detecting ER protein (contacting the sample with an ER-specific antibody and detecting ER protein in the sample), and then followed by detecting HER2 genomic DNA (contacting the sample with a HER2 genomic DNA-specific nucleic acid probe and detecting HER2 genomic DNA).

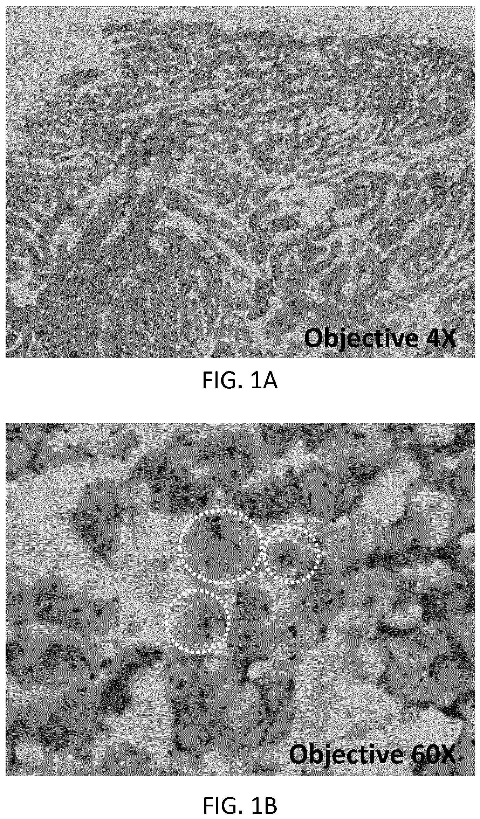

As an example, reference is made to FIGS. 1A-B, showing a pair of images of a breast tumor tissue sample stained for HER2 gene (black punctate nuclear staining), HER2 protein (brown membrane staining), and ER protein (red cytoplasmic staining) at 4X magnification (FIG. 1A) and 60X magnification (FIG. 1B). The sample is HER2 gene amplified, HER2 protein positive, and ER protein positive. However, some cells (circled) are negative for HER2 protein, though they are ER protein positive and have HER2 gene amplification. Since the HER2-targeted therapies target the HER2 protein, this heterogeneity could result in failure of the therapy to affect (e.g., inhibit or even kill) tumor cells that are HER2 gene amplified, but do not overexpress the HER2 protein. However, those cells that are ER-positive would still be affected by ER-targeted therapies.

In additional embodiments the method includes simultaneously contacting the sample with a HER2 genomic DNA-specific nucleic acid probe and a chromosome 17 centromere genomic DNA-specific nucleic acid probe and detecting HER2 genomic DNA and then detecting chromosome 17 centromere genomic DNA.

In some examples of the disclosed methods, the sample is contacted with an antibody that specifically binds to HER2 protein. Methods of constructing HER2-specific antibodies are known in the art. In addition, such antibodies may be commercially available. In one specific example, the sample is contacted with an anti-HER2 rabbit monoclonal antibody, such as anti-HER-2/neu (4B5) rabbit monoclonal antibody (Ventana Medical Systems, Inc., Tucson, AZ, e.g., catalog number 790-2991). Additional HER2-specific antibodies include anti-c-erbB2 antibody A0485 (Dako, Carpinteria, CA). In some examples, the HER2-specific antibody is detectably labeled, allowing detection of HER2 protein in the sample. In other examples, after contacting the sample with the anti-HER2 antibody (the primary antibody), the sample is contacted with a detectably labeled secondary antibody raised against the primary antibody, such as a secondary antibody conjugated to an enzyme (for example, alkaline phosphatase (AP) or horseradish peroxidase (HRP)) or a secondary antibody conjugated to a hapten that can be detected with a further reagent conjugated to an enzyme. The presence of HER2 protein is detected by contacting the enzyme with a chromogen and/or substrate composition which produces a colored precipitate in the vicinity of the anti-HER2 antibody. The presence and/or amount of HER2 protein is detected by determining staining intensity in the sample. In some examples, the staining intensity is rated by a slide reader on a numeric scale, such as a scale of 0-3 (for example, where 0 indicates no staining relative to background, 1 indicates weak staining, 2 indicates moderate staining, and 3 indicates strong staining).

In one particular example, the method includes contacting the sample with a primary antibody that specifically binds to the HER2 protein (for example, anti-HER2 4B5 rabbit monoclonal antibody), for example under conditions sufficient for the anti-HER2 antibody to specifically bind to HER2 protein in the sample. The sample is then contacted with a biotinylated secondary antibody that specifically binds the primary antibody, for example under conditions sufficient for the secondary antibody to specifically bind to the primary antibody. The sample is then contacted with HRP- conjugated streptavidin, for example under conditions sufficient for the streptavidin-HRP to specifically bind to the biotin, followed by contacting the sample with hydrogen peroxide substrate and 3,3'-diaminobenzidine (DAB) chromogen, which produces a brown precipitate near the anti-HER2 antibody (and HER2 protein) that can be visually detected by light (bright-field) microscopy. In one example, the reagents (except for the anti-HER2 antibody) are included in a kit, such as the IVIEW DAB Detection Kit (Ventana Medical Systems, Tucson, AZ, catalog number 760-091). One of ordinary skill in the art can select alternative detection reagents (such as alternative secondary antibodies, enzymes, substrates, and/or chromogens) including those that produce a different color precipitate for detection of the HER2 protein.

In some examples of the disclosed methods, the sample is contacted with an antibody that specifically binds to ER protein. Methods of constructing ER-specific antibodies are known in the art. In addition, such antibodies may be commercially available. In one specific example, the sample is contacted with an anti-ER rabbit monoclonal antibody, such as anti-ER (SP1) rabbit monoclonal antibody (Ventana Medical Systems, Inc., Tucson, AZ, e.g., catalog number 790-4324). Additional ER- specific antibodies include anti-ER monoclonal antibodies 1D5 and ER-2-123 (Dako, Carpinteria, CA). In some examples, the ER-specific antibody is detectably labeled, allowing detection of ER protein in the sample. In other examples, after contacting the sample with the anti-ER antibody (the primary antibody), the sample is contacted with a detectably labeled secondary antibody raised against the primary antibody, such as a secondary antibody conjugated to an enzyme (for example, AP or HRP) or a secondary antibody conjugated to a hapten that can be detected with a further reagent conjugated to an enzyme. The presence of ER protein is detected by contacting the enzyme with a chromogen and/or substrate composition, which produces a colored precipitate in the vicinity of the anti-ER antibody. The presence and/or amount of ER protein is detected by determining staining intensity in the sample. In some examples, the staining is scored by a slide reader by determining a percentage of tumor cells in the sample that are stained for the ER protein.

In one particular example, the method includes contacting the sample with a primary antibody that specifically binds to the ER protein (for example, anti-ER SP1 rabbit monoclonal antibody), for example under conditions sufficient for the anti-ER antibody to specifically bind to ER protein in the sample. The sample is then contacted with an AP-conjugated secondary antibody that specifically binds the primary antibody, for example under conditions sufficient for the secondary antibody to specifically bind to the primary antibody. The sample is then contacted with a naphthol phosphate and Fast Red chromogen, which produces a red precipitate near the anti-ER antibody (and ER protein) that can be visually detected by light microscopy. In one example, the reagents (except for the anti-ER antibody) are included in a kit, such as the ULTRAVIEW Universal Alkaline Phosphatase Red Detection Kit (Ventana Medical Systems, Tucson, AZ, catalog number 760-501). One of ordinary skill in the art can select alternative detection reagents (such as alternative antibodies, enzymes, substrates, and/or chromogens) including those that produce a different color precipitate for detection of the ER protein.

Alternatively, the method includes contacting the sample with a primary antibody that specifically binds to the ER protein (for example, anti-ER SP1 rabbit monoclonal antibody), for example under conditions sufficient for the anti-ER antibody to specifically bind to ER protein in the sample. The sample is then contacted with a biotinylated secondary antibody that specifically binds the primary antibody, for example under conditions sufficient for the secondary antibody to specifically bind to the primary antibody. The sample is then contacted with streptavidin-HRP, followed by hydrogen peroxide and Discovery Purple chromogen (a tyramide-rhodamine conjugate; Ventana Medical Systems, Tucson, AZ, part number 700-229), which produces a purple dye bound to the sample near the anti-ER antibody (and ER protein) that can be visually detected by light microscopy.

In some examples, of the disclosed methods, the sample is contacted with a nucleic acid probe that specifically binds to HER2 genomic DNA. Methods of constructing HER2-specific nucleic acid probes are known to one of ordinary skill in the art. HER2-specific nucleic acid probes may also be commercially available. For example, a HER2 probe suitable for use in the disclosed methods includes the HER2 probe included in the INFORM HER2 Dual ISH Probe Cocktail (Ventana Medical Systems, Tucson, AZ, catalog number 780-4422). In one example, the sample is contacted with a hapten-labeled HER2 nucleic acid probe, for example under conditions specific for the probe to specifically bind to (hybridize with) HER2 genomic DNA in the sample. The sample is then contacted with an antibody that specifically binds to the hapten, for example, under conditions sufficient for the antibody to specifically bind to the hapten. The antibody may be conjugated to an enzyme (such as AP or HRP) or alternatively, the sample may be contacted with a second antibody that specifically binds the anti-hapten antibody, where the second antibody is conjugated to an enzyme. The presence of HER2 genomic DNA is detected by contacting the enzyme with a chromogen and/or substrate composition to produce a colored precipitate in the vicinity of the HER2 nucleic acid probe. In some examples, the gene copy number of HER2 DNA in the sample is scored by a slide reader by counting the number of areas of precipitate ("spots") in the nuclei of the tumor cells.

In one particular example, the method includes contacting the sample with a HER2 genomic DNA probe conjugated to dinitrophenyl (DNP), for example under conditions sufficient for the HER2 probe to specifically bind to HER2 genomic DNA in the sample. The sample is then contacted with an anti-hapten antibody that specifically binds DNP, for example under conditions sufficient for the anti-DNP antibody to specifically bind to the DNP. The sample is then contacted with an HRP-conjugated secondary antibody that specifically binds to the anti-DNP antibody, for example under conditions sufficient for the secondary antibody to specifically bind to the anti-DNP antibody. The sample is then contacted with chromogen and substrate silver acetate, hydroquinone, and hydrogen peroxide. The silver ions are reduced by hydroquinone to metallic silver ions which can be visually detected by light microscopy as black spots. In one example, the reagents (except for the HER2 probe) are included in a kit, such as the ULTRA VIEW SISH DNP Detection Kit (Ventana Medical Systems, Tucson, AZ, catalog number 760-098). One of ordinary skill in the art can select alternative detection reagents (such as alternative haptens, antibodies, enzymes, substrates, and/or chromogens) including those that produce a different color precipitate for detection of HER2 genomic DNA.

In additional examples, the disclosed methods further include contacting the sample with a probe that specifically binds to chromosome 17 centromere DNA and detecting chromosome 17 DNA (such as chromosome 17 copy number) in the sample. In some examples of the disclosed methods, the sample is contacted with a nucleic acid probe that specifically binds to chromosome 17 centromere DNA. Methods of constructing chromosome 17 centromere-specific nucleic acid probes are known to one of ordinary skill in the art. In addition, chromosome 17 centromere nucleic acid probes may also be commercially available. For example, a chromosome 17 centromere probe suitable for use in the disclosed methods includes the chromosome 17 centromere probe included in the INFORM HER2 Dual ISH Probe Cocktail (Ventana Medical Systems, Tucson, AZ, catalog number 780-4422). In one example, the sample is contacted with a hapten-labeled chromosome 17 centromere nucleic acid probe, for example under conditions specific for the probe to specifically bind to (hybridize with) chromosome 17 centromere genomic DNA in the sample. The sample is then contacted with an antibody that specifically binds to the hapten, for example, under conditions sufficient for the antibody to specifically bind to the hapten. The antibody may be conjugated to an enzyme (such as AP or HRP) or alternatively, the sample may be contacted with a second antibody that specifically binds the anti-hapten antibody, where the second antibody is conjugated to an enzyme. The presence of chromosome 17 centromere genomic DNA is detected by contacting the enzyme with a chromogen and/or substrate composition to produce a colored precipitate in the vicinity of the chromosome 17 centromere nucleic acid probe. In some examples, the gene copy number of chromosome 17 centromere DNA in the sample is scored by a slide reader by counting the number of areas of precipitate ("spots") in the nuclei of the tumor cells.

In a particular example, the method includes contacting the sample with a chromosome 17 centromere DNA probe conjugated to digoxigenin (DIG), for example under conditions sufficient for the chromosome 17 centromere probe to specifically bind to chromosome 17 centromere DNA in the sample. The sample is then contacted with an anti-hapten antibody that specifically binds DIG, for example under conditions sufficient for the anti-DIG antibody to specifically bind to the DIG. The sample is then contacted with an AP-conjugated secondary antibody that specifically binds to the anti- DIG antibody, for example under conditions sufficient for the secondary antibody to specifically bind to the anti-DIG antibody. The sample is then contacted with a naphthol phosphate and Fast Red, producing a red precipitate which is deposited in the nuclei near the chromosome 17 centromere probe (and the chromosome 17 centromere DNA) and can be visually detected by light microscopy as red spots. In one example, the reagents (except for the chromosome 17 centromere probe) are included in a kit, such as the ULTRAVIEW Red ISH DIG Detection Kit (Ventana Medical Systems, Tucson, AZ, catalog number 760-505). One of ordinary skill in the art can select alternative detection reagents (such as alternative haptens, antibodies, enzymes, substrates, and/or chromogens) including those that produce a different color precipitate for detection of chromosome 17 centromere DNA.

The disclosed methods are directed to detection of multiple protein and nucleic acid targets in a single sample. As a result, the detectable signal for each member of the assay must be individually distinguishable. Therefore, in some examples, the visual signal generated by the detection assay for each member of the assay is a different color. In one specific example, the methods result in a brown staining for HER2 protein (for example, brown staining at the cell membrane), red staining for ER protein (for example red staining in the nucleus), and black staining for HER2 genomic DNA (for example, black spots in the nucleus, such as individually distinguishable black spots or clusters of black spots). In another specific example, the methods result in a brown staining for HER2 protein, purple staining for ER protein, and black staining for HER2 genomic DNA. One of ordinary skill in the art can select different combinations of detection reagents to provide different colored staining for each of the HER2 protein, ER protein, and HER2 genomic DNA. In additional examples, the methods further result in red staining for chromosome 17 centromere DNA (for example, red spots in the nucleus, such as individually distinguishable red spots or clusters of red spots). In a particular example, the methods result in brown staining of HER2 protein, purple staining of ER protein, black staining of HER2 genomic DNA, and red staining of chromosome 17 centromere DNA. In some embodiments, HER2 protein staining with DAB (brown) staining is utilized because this is the currently accepted detection system and is familiar to pathologists. However, additional color combinations can be used.

The methods disclosed herein may also include steps for pre-treatment of tissue samples prior to or between the steps including contacting the sample with a HER2-specific antibody, and ER-specific antibody, a HER2-specific nucleic acid probe, and/or a chromosome 17 centromere-specific nucleic acid probe. These steps are known to one of ordinary skill in the art and may include deparaffinization of a sample (such as a FFPE sample), cell conditioning, washes, and so on. An exemplary protocol, including such pre-treatment and other steps is provided in Example 1. One of skill in the art can make adjustments to these conditions (for example, minor adjustments to times and/or temperatures of incubations, wash steps, etc.).

Exemplary chromogens that can be used in the disclosed methods include (but are not limited to) those shown in Table 1. While not exhaustive, Table 1 provides insight into the varieties of presently available chromogens. Further illustrative chromogens include those described in U.S. Pat. Publ. 2013/0260379 and U.S. Prov. Pat. App. No. 61/831,552, filed June 5, 2013.

**Table 1: Chromogenic detection reagents.**

| **Abbr.** | **Name** | **Color** | **Enzyme** |
|---|---|---|---|
| DAB | 3,3'-diamino-benzidine + H₂O₂ | brown - black | peroxidase |
| AEC | 3-amino-9-ethyl-carbazole + H₂O₂ | red | peroxidase |
| CN | 4-chloro-1-naphthol+H₂O₂ | blue | peroxidase |
| BCIP/NBT | 5-bromo-4-chloro-3-indolyl-phosphate + nitroblue tetrazolium | indigo - black | alkaline phosphatase |
| FAST RED | 4-chloro-2-methylbenzenediazonium + 3-hydroxy-2-naphthoic acid 2,4-dimethylanilide phosphate | red | alkaline phosphatase |
| FAST BLUE | Naphthol AS-MX phosphate disodium salt + fast blue BB salt hemi(zinc chloride) salt | blue | alkaline phosphatase |
| FUCHSIN | Naphthol AS-BI + New Fuchsin | red | alkaline phosphatase |
| NBT | nitroblue tetrazolium + phenazine methosulfate | blue -purple | dehydrogenase |
| ALK GOLD† | 3-methyl-1-phenyl-1H-pyrazol-5-yl dihydrogen phosphate + fast blue BB | yellow - gold | alkaline phosphatase |

Table 1, while not exhaustive, provides insight into the varieties of presently available chromogenic substances (†WO2012/024185, Kelly et al. "Substrates for Chromogenic detection and methods of use in detection assays and kits").

In some embodiments, the methods include determining whether the sample is positive or negative for HER2. In some examples, the sample is determined to be positive or negative for HER2 protein, positive or negative for HER2 gene amplification, or both. One of ordinary skill in the art can determine whether a sample (such as a breast tumor sample) is positive or negative for HER2 protein and/or HER2 gene amplification. In some examples, the sample is scored semi-quantitatively for HER2 protein, such as 0 (negative), 1+ (negative), 2+ (equivocal), or 3+ (positive). In some examples, the sample is scored for HER2 gene amplification based on HER2 gene copy number, such as six or more copies of HER2 (positive) or fewer than six copies of HER2 (negative). In other examples, the sample is scored for HER2 gene amplification based on the ratio of HER2 gene copy number to chromosome 17 centromere copy number, such as HER2/CEN17<1.8 (negative), 1.8≥HER2/CEN17≤2.2 (equivocal), HER2/CEN17>2.2 (positive). Additional HER2 test guidelines are available and include those described in Wolff et al., J. Clin. Oncol., doi:10.1200/JCO.2013.50.9984.

In some embodiments, the methods also include determining whether the sample is positive or negative for ER protein. One of ordinary skill in the art can determine whether a sample (such as a breast tumor sample) is positive or negative for ER protein. In some examples, a sample is determined to be ER positive if there is ER protein staining in the nucleus of ≥1% of the tumor cells in the sample and is determined to be ER negative if there is ER protein staining in the nucleus of <1% of the tumor cells in the sample. In additional examples, a sample is determined to have low ER expression if ER staining is detected in 1-10% of tumor cells in the sample and is determined to have high ER expression if ER staining is detected in >10% of the tumor cells in the sample.

The disclosed methods can be automated (for example, as described in Example 1). Systems for automated IHC and/or ISH are commercially available, such as the BENCHMARK ULTRA slide staining system, the BENCHMARK XT slide staining system, and the DISCOVERY XT slide staining system (Ventana Medical Systems, Tucson, AZ), BOND-MAX and BOND-III slide stainers (Leica Biosystems, Buffalo Grove, IL), and the IQ Kinetic slide stainer (Biocare Medical, Concord, CA). Ventana Medical Systems, Inc. is the assignee of a number of United States patents disclosing systems and methods for performing automated analyses, including U.S. Patent Nos. 5,650,327; 5,654,200; 6,296,809; 6,352,861; 6,582,962; 6,827,901 and 6,943,029.

### III. Samples

Exemplary samples include, without limitation, blood smears, cytocentrifuge preparations, cytology smears, core biopsies, and/or fine-needle aspirates. In some examples, the samples include tissue sections (e.g., cryostat tissue sections and/or paraffin-embedded tissue sections). In particular embodiments, the samples include tumor cells, such as breast tumor cells or ovarian tumor cells. Methods of obtaining a biological sample from a subject are known in the art. For example, methods of obtaining breast tissue or breast cells are routine. Exemplary biological samples may be isolated from normal cells or tissues, or from neoplastic cells or tissues. In particular examples, a biological sample includes a tumor sample, such as a breast tumor sample.

For example, a sample from a breast tumor that contains cellular material can be obtained by surgical excision of all or part of the tumor, by collecting a fine needle aspirate from the tumor, as well as other methods known in the art. In particular examples, a tissue or cell sample is applied to a substrate and analyzed to detect HER2 protein, ER protein, and HER2 genomic DNA. A solid support can hold the biological sample and permit the convenient detection of components (e.g., proteins and/or nucleic acid molecules) in the sample. Exemplary supports include microscope slides (e.g., glass microscope slides or plastic microscope slides), coverslips (e.g., glass coverslips or plastic coverslips), tissue culture dishes, multi-well plates, membranes (e.g., nitrocellulose or polyvinylidene fluoride (PVDF)) or BIACORE™ chips.

The samples described herein can be prepared using any method now known or hereafter developed in the art. Generally, tissue samples are prepared by fixing and embedding the tissue in a medium. In other examples, samples include a cell suspension which is prepared as a monolayer on a solid support (such as a glass slide) for example by smearing or centrifuging cells onto the solid support. In further examples, fresh frozen (for example, unfixed) tissue sections may be used in the methods disclosed herein.

The process of fixing a sample can vary. Fixing a tissue sample preserves cells and tissue constituents in as close to a life-like state as possible and allows them to undergo preparative procedures without significant change. Fixation arrests the autolysis and bacterial decomposition processes that begin upon cell death, and stabilizes the cellular and tissue constituents so that they withstand the subsequent stages of tissue processing, such as for ISH or IHC.

Tissues can be fixed by any suitable process, including perfusion or by submersion in a fixative. Fixatives can be classified as cross-linking agents (such as aldehydes, e.g., formaldehyde, paraformaldehyde, and glutaraldehyde, as well as non-aldehyde cross-linking agents), oxidizing agents (e.g., metallic ions and complexes, such as osmium tetroxide and chromic acid), protein-denaturing agents (e.g., acetic acid, methanol, and ethanol), fixatives of unknown mechanism (e.g., mercuric chloride, acetone, and picric acid), combination reagents (e.g., Carnoy's fixative, methacarn, Bouin's fluid, B5 fixative, Rossman's fluid, and Gendre's fluid), microwaves, and miscellaneous fixatives (e.g., excluded volume fixation and vapor fixation). Additives may also be included in the fixative, such as buffers, detergents, tannic acid, phenol, metal salts (such as zinc chloride, zinc sulfate, and lithium salts), and lanthanum.

The most commonly used fixative in preparing samples is formaldehyde, generally in the form of a formalin solution (4% formaldehyde in a buffer solution, referred to as 10% buffered formalin). In one example, the fixative is 10% neutral buffered formalin.

In some examples an embedding medium is used. An embedding medium is an inert material in which tissues and/or cells are embedded to help preserve them for future analysis. Embedding also enables tissue samples to be sliced into thin sections. Embedding media include paraffin, celloidin, OCT™ compound, agar, plastics, or acrylics. Many embedding media are hydrophobic; therefore, the inert material may need to be removed prior to histological or cytological analysis, which utilizes primarily hydrophilic reagents. The term deparaffinization or dewaxing is broadly used herein to refer to the partial or complete removal of any type of embedding medium from a biological sample. For example, paraffin-embedded tissue sections are dewaxed by passage through organic solvents, such as toluene, xylene, limonene, or other suitable solvents.

### IV. Methods of Treatment

The disclosed methods can further include selecting and/or administering a treatment to the subject. In some examples, a treatment is selected and administered based on the HER2 and/or ER status of the subject's tumor. For example, a subject with an ER positive/HER2 negative tumor is administered one or more anti-estrogen therapeutics, such as tamoxifen, letrozole, toremifene, fulvestrant, anastrozole, and/or exemestane. A subject with a HER2 positive/ER negative tumor is administered one or more HER2-targeting therapies, such as trastuzumab, lapatinib, pertuzumab, and/or trastuzumab emtansine. A subject with a HER2 positive/ER positive tumor is administered both one or more anti-estrogen therapeutics and one or more HER2- targeting therapies. In one example, a subject with a HER2 positive/ER positive tumor is administered trastuzumab and letrozole; trastuzumab and anastrozole; or trastuzumab, lapatinib, and letrozole. In additional examples, subjects are also administered neoadjuvant chemotherapy, regardless of ER or HER2 status. For example, subjects can be treated with taxanes (such as paclitaxel or docetaxel), anthracyclines (such as daunorubicin, doxorubicin, epirubicin, or mitoxantrone), cyclophosphamide, capecitabine, 5-fluorouracil, methotrexate, or combinations thereof. One of skill in the art can select appropriate therapeutic regimens for a subject based on the HER2 and ER status of the subject, and the age, condition, previous treatment history of the subject, and other factors.

The following examples are provided to illustrate certain specific features of working embodiments and general protocols. The scope of the present disclosure is not limited to those features exemplified by the following examples.

### Example 1

### HER2 and ER Gene-Protein Assay

This example describes a multiplex gene-protein assay for detection of HER2 protein, ER protein, and HER2 gene copy number in a sample.

A multiplex assay for detection of HER2 and ER protein and HER2 gene copy number in a single sample was developed. HER2 protein was first detected by IHC using PATHWAY anti-HER2/neu (4B5) rabbit monoclonal antibody (Ventana Medical Systems, Tucson, AZ) with iVIEW DAB detection (Ventana Medical Systems, Tucson, AZ). ER protein was next detected by IHC using CONFIRM anti-estrogen receptor (SP1) rabbit monoclonal antibody (Ventana Medical Systems, Tucson, AZ) with ULTRA VIEW Universal DAB detection (Ventana Medical Systems, Tucson, AZ). Finally, HER2 genomic DNA was detected with ISH using a DNP-labeled HER2 probe and detected with ULTRAVIEW SISH DNP detection (Ventana Medical Systems, Tucson, AZ). All steps were performed on a BENCHMARK XT automated IHC/ISH staining instrument (Ventana Medical Systems, Tucson, AZ, Catalog #: N750-BMKXT- FS) with NexES V10.6 as follows:
(1) Baking: 60°C for 4 minutes, rinse;
(2) Deparaffinization was performed to remove the wax for reagent penetration using EZ Prep (VMSI Catalog #: 950-102): 2x8 minutes at 72°C, rinse;
(3) Cell Conditioning was performed using used CC1 (VMSI Catalog #: 950-124) 2x16 minutes and 1x8 minutes at 95°C, rinse slide with reaction buffer;
(4) Treat with IVIEW inhibitor (VMSI Catalog #: 253-2187) for 4 minutes at 37°C, rinse slide with reaction buffer;
(5) Primary Antibody Application: PATHWAY anti-HER2/neu 4B5 antibody (VMSI Catalog #790-2991), incubated for 32 minutes at 37°C, rinse slide with reaction buffer;
(6) Detection with IVIEW DAB Detection system: Biotin Blocker A (VMSI catalog #253-2030) for 4 minutes at 37°C, rinse, Biotin Blocker B (VMSI catalog #253-2031) for 4 minutes at 37°C, rinse, IVIEW biotin Ig (VMSI catalog #253-2188) for 8 minutes at 37°C, rinse, IVIEW SA-HRP (VMSI catalog #253-2189) for 8 minutes at 37°C, rinse, IVIEW DAB (VMSI catalog #253-2190) and IVIEW hydrogen peroxide (VMSI catalog #253-2191) for 8 minutes at 37°C, rinse, and IVIEW Copper (VMSI catalog #253-2192) for 4 minutes at 37°C, rinse (all rinses with reaction buffer);
(7) Reaction buffer was applied and the sample was incubated at 95°C for 8 minutes, incubated 4 minutes without heating, rinsed with reaction buffer
(8) Primary Antibody Application: CONFIRM anti-ER (SP1) antibody (VMSI catalog #790-4324), incubated for 16 minutes at 37°C, rinse slide with reaction buffer;
(9) Detection was with ULTRAVIEW Universal Alkaline Phosphatase Red Detection System: ULTRAVIEW Red Universal Alkaline Phosphatase Multimer (VMSI catalog #253-4327) for 16 minutes at 37°C, rinse, ULTRAVIEW Red enhancer (VMSI catalog #253-4326) for 4 minutes at 37°C, ULTRAVIEW Red naphthol (VMSI catalog #253-4328) for 4 minutes at 37°C, ULTRAVIEW Fast Red A (VMSI catalog #253-429) and ULTRAVIEW Fast Red B (VMSI catalog #253-4330) for 16 minutes at 37°C, rinse (all rinses with reaction buffer);
(10) Apply 900 µl of rinse buffer, 4 minutes at 37°C, Cell Conditioning: Cell Conditioner 2 (VMSI catalog #950-123) for 3 cycles of 8 minutes at 90°C, rinse;
(11) Protease treatment: ISH Protease 2 (VMSI catalog #780-4148) for 12 minutes at 37°C, rinse;
(12) Clarification: HybClear solution (VMSI catalog #780-4572) for 4 minutes at 52°C;
(13) Probe: HER2 DNP probe(VMSI catalog #780-4422) for 4 minutes at 52°C, 4 minutes at 80°C, and 6 hours at 44°C, rinse;
(14) Stringency wash with rinse buffer 4x8 minutes at 72°C, rinse
(15) Detection with ULTRAVIEW SISH DNP Detection system: silver ISH anti-DNP antibody (VMSI catalog #253-4414) for 20 minutes at 37°C, rinse, silver ISH DNP HRP (VMSI catalog #253-4413) for 24 minutes at 37°C, rinse, silver ISH DNP chromogen A (VMSI catalog #253-4410) for 4 minutes at room temperature, rinse, silver ISH DNP chromogen A for 4 minutes at room temperature, silver ISH DNP chromogen B(VMSI catalog #253-4411) for 4 minutes at room temperature, and silver ISH DNP chromogen C (VMSI catalog #253-4412) for 8 minutes at room temperature, rinse;
(16) Counterstain & Post-counterstain: 8 minutes with Hematoxylin II (VMSI Catalog #: 790-2208), rinse, Post-counterstain 4 minutes with Bluing Reagent (VMSI Catalog #: 760-2037).

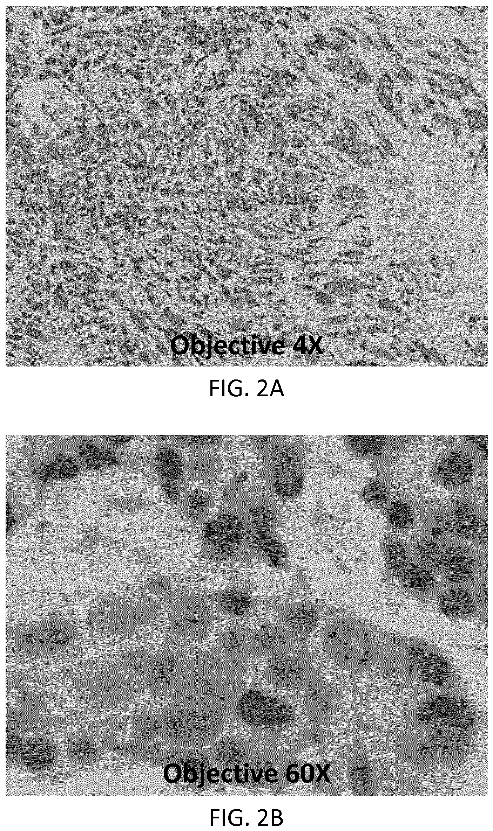

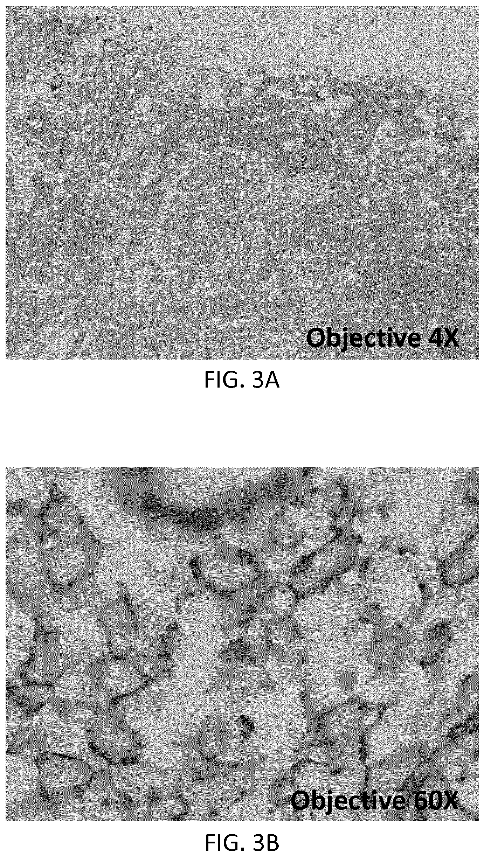

The staining protocol results in brown staining of HER2 protein, red staining of the ER protein, and black staining of the HER2 genomic DNA. Representative breast tumor samples showing a sample which has amplified HER2 gene, is HER2 protein positive and ER protein positive (FIGS. 1A and B), a sample with amplified HER2 gene, HER2 protein negative, and ER protein positive (FIGS. 2A and B), and a sample with amplified HER2 gene, HER2 protein positive, and ER protein negative (FIGS. 3A and B) are provided. Within sample heterogeneity was observed. For example, even in the HER2 protein positive sample (FIG. 1), some cells were HER2 gene amplification and ER protein positive, but lacked HER2 protein (circled cells in FIG. 1B).

### Example 2

### Comparison of Detection Methods and Use of Ki67

This example describes comparison of detection methods for the ER protein IHC and also comparison of ER IHC with Ki67 IHC.

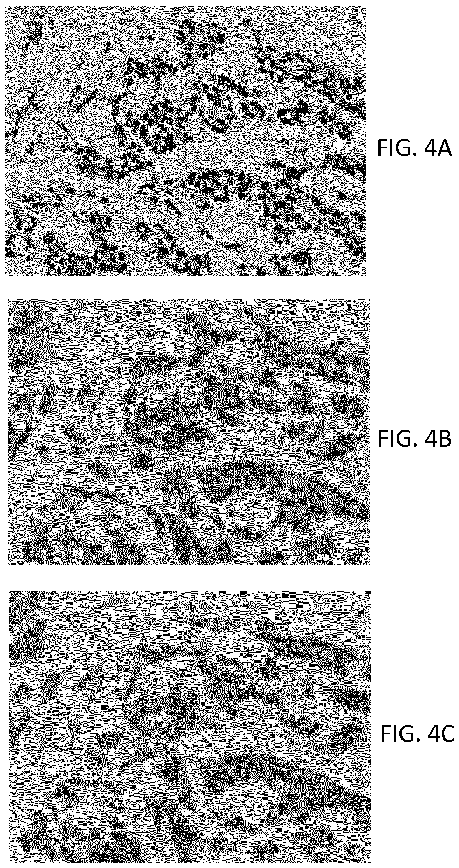

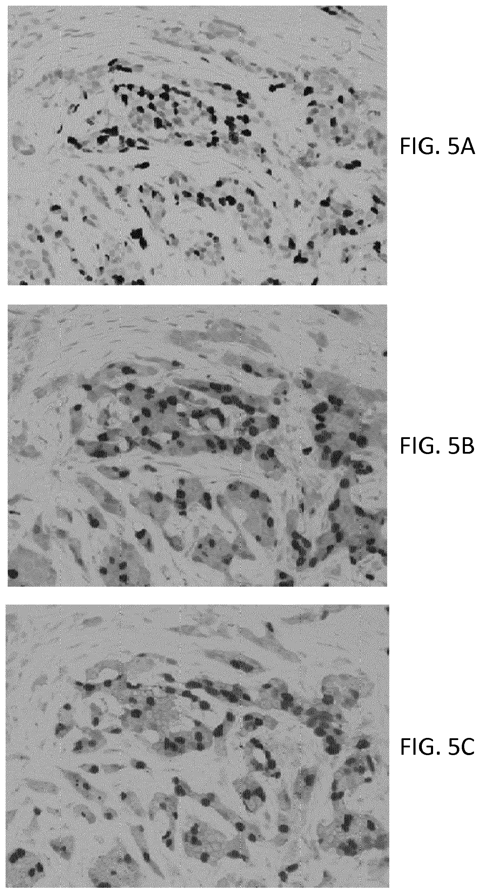

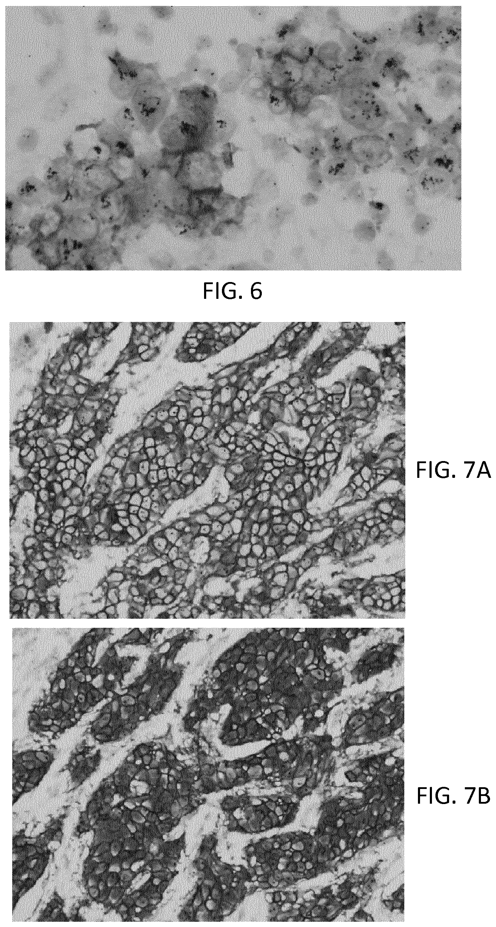

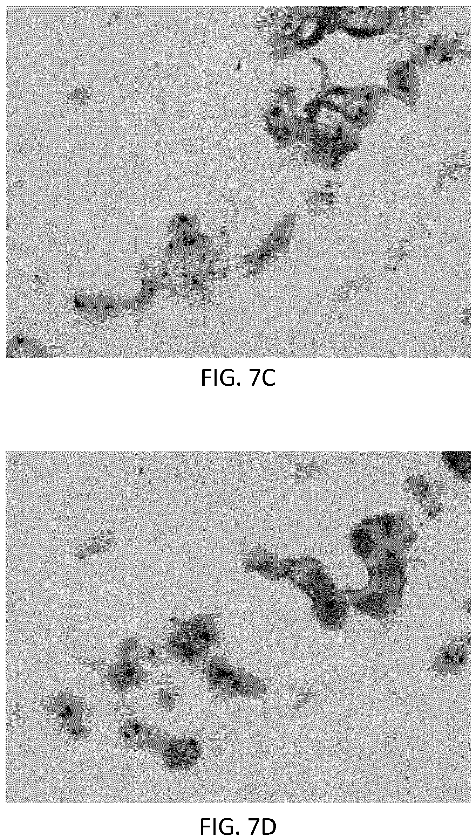

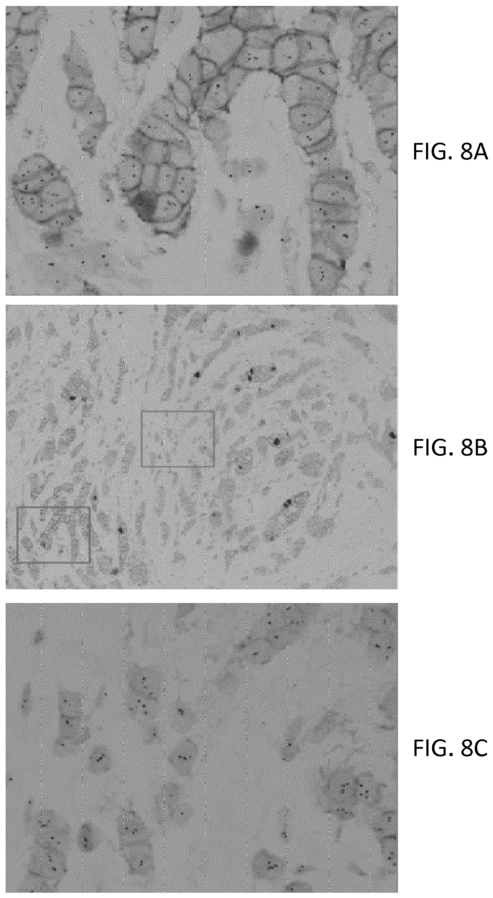

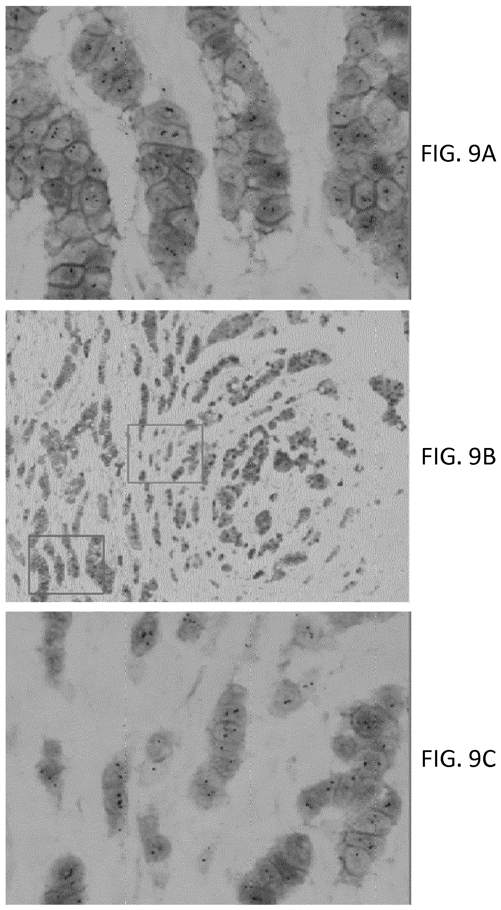

Staining of ER protein IHC with iVIEW DAB reagents or ULTRAVIEW Red reagents was tested in breast tumor samples (FIGS. 4A and B) and compared with the HER2 IHC/ISH stained with ULTRAVIEW Red (FIG. 4C). The ULTRAVIEW Red staining (FIG. 4C) was selected for inclusion in the assay (as described in Example 1). Similar experiments were performed using Ki67 protein IHC instead of ER IHC (FIGS. 5A-C). FIG. 6 shows a sample stained for HER2 gene, HER2 protein, and Ki67 protein. An example of HER2 gene and protein staining with Ki67 or ER IHC in a HER2 positive sample is shown in FIGS. 7A-D. An example of HER2 gene and protein staining with Ki67 or ER IHC in an HER2 equivocal case is shown in FIGS. 8 and 9, respectively.

### Example 3

### Fourplex HER2 and ER Gene-Protein Assay

This example describes a multiplex gene-protein assay for detection of HER2 protein, ER protein, HER2 gene copy number, and chromosome 17 copy number in a sample.

A multiplex assay for detection of HER2 and ER protein, HER2 gene copy number, and chromosome 17 centromere DNA gene copy number in a single sample was developed. HER2 protein was first detected by IHC using PATHWAY anti-HER2/neu (4B5) rabbit monoclonal antibody (Ventana Medical Systems, Tucson, AZ) with iVIEW DAB detection (Ventana Medical Systems, Tucson, AZ). ER protein was next detected by IHC using CONFIRM anti-estrogen receptor (SP1) rabbit monoclonal antibody (Ventana Medical Systems, Tucson, AZ) with Discovery Purple detection (Ventana Medical Systems, Tucson, AZ). Finally HER2 nucleic acid genomic DNA and chromosome 17 centromere DNA were detected with dual ISH using a DNP-labeled HER2 probe detected with ULTRAVIEW SISH DNP detection (Ventana Medical Systems, Tucson, AZ) and a DIG-labeled chromosome 17 centromere probe detected with ULTRAVIEW Red ISH DIG detection (Ventana Medical Systems, Tucson, AZ). All steps were performed on a BENCHMARK XT automated IHC/ISH staining instrument (Ventana Medical Systems, Tucson, AZ, Catalog #: N750-BMKXT- FS) with NexES V10.6 as follows:
(1) Baking: 60°C for 4 minutes, rinse;
(2) Deparaffinization was performed to remove the wax for reagent penetration using EZ Prep (VMSI Catalog #: 950-102): 2x8 minutes at 72°C, rinse;
(3) Cell Conditioning was performed using used CC1 (VMSI Catalog #: 950-124) 2x16 minutes and 1x8 minutes at 95°C, rinse slide with reaction buffer;
(4) Treat with IVIEW inhibitor (VMSI Catalog #: 253-2187) for 4 minutes at 37°C, rinse slide with reaction buffer;
(5) Primary Antibody Application: PATHWAY anti-HER2/neu 4B5 antibody (VMSI Catalog #790-2991), incubated for 32 minutes at 37°C, rinse slide with reaction buffer;
(6) Detection with IVIEW DAB Detection system: Biotin Blocker A (VMSI
   catalog #253-2030) for 4 minutes at 37°C, rinse, Biotin Blocker B (VMSI catalog #253-2031) for 4 minutes at 37°C, rinse, IVIEW biotin Ig (VMSI catalog #253-2188) for 8 minutes at 37°C, rinse, IVIEW SA-HRP (VMSI catalog #253-2189) for 8 minutes at 37°C, rinse, IVIEW DAB (VMSI catalog #253-2190) and IVIEW hydrogen peroxide (VMSI catalog #253-2191) for 8 minutes at 37°C, rinse, and IVIEW Copper (VMSI catalog #253-2192) for 4 minutes at 37°C, rinse (all rinses with reaction buffer);
(7) Reaction buffer was applied and the sample was incubated at 95°C for 8 minutes, incubated 4 minutes without heating, rinsed with reaction buffer;
(8) Primary Antibody Application: CONFIRM anti-ER (SP1) antibody (VMSI catalog #790-4324), incubated for 16 minutes at 37°C, rinse slide with reaction buffer;
(9) Detection: IVIEW biotin Ig (VMSI catalog #253-2188) for 8 minutes at 37°C, rinse, IVIEW SA-HRP (VMSI catalog #253-2189) for 8 minutes at 37°C, rinse, Discovery Purple (VMSI catalog #700-229) and hydrogen peroxide for 32 minutes at 37°C, rinse (all rinses with reaction buffer);
(10) Apply 900 µl of rinse buffer, 4 minutes at 37°C, Cell Conditioning: Cell Conditioner 2 (VMSI catalog #950-123) for 3 cycles of 8 minutes at 90°C, rinse;
(11) Protease treatment: ISH Protease 2 (VMSI catalog #780-4148) for 8 minutes at 37°C, rinse;
(12) Clarification: HybClear solution (VMSI catalog #780-4572) for 4 minutes at 52°C;
(13) Probe: HER2 DNP and Chr17 DIG probe cocktail (VMSI catalog #780-4422) for 4 minutes at 52°C, 4 minutes at 80°C, and 6 hours at 44°C, rinse;
(14) Stringency wash with rinse buffer 4x8 minutes at 72°C, rinse;
(15) HER2 Detection with ULTRAVIEW SISH DNP Detection system: silver ISH anti-DNP antibody (VMSI catalog #253-4414) for 20 minutes at 37°C, rinse, silver ISH DNP HRP (VMSI catalog #253-4413) for 24 minutes at 37°C, rinse, silver ISH DNP chromogen A (VMSI catalog #253-4410) for 4 minutes at room temperature, rinse, silver ISH DNP chromogen A for 4 minutes at room temperature, silver ISH DNP chromogen B(VMSI catalog #253-4411) for 4 minutes at room temperature, and silver ISH DNP chromogen C (VMSI catalog #253-4412) for 8 minutes at room temperature, rinse (all rinses with reaction buffer);
(16) Chr17 Detection with ULTRAVIEW Red ISH DIG detection system: ULTRAVIEW Red ISH DIG mouse anti-DIG antibody (VMSI catalog #253-4415) for 20 minutes at 37°C, rinse, ULTRAVIEW Red ISH DIG AP (VMSI catalog #253-4419) for 32 minutes at 37°C, rinse, ULTRAVIEW Red ISH DIG pH Enhancer (VMSI catalog #253-4418) for 8 minutes at 37°C, ULTRAVIEW Red ISH DIG naphthol (VMSI catalog #253-4417) for 4 minutes at 37°C, ULTRAVIEW Red ISH DIG Fast Red (VMSI catalog #253-4416) for 4 minutes, ULTRAVIEW Red ISH DIG Fast Red for 12 minutes at 37°C, rinse (all rinses with reaction buffer);
(17) Counterstain & Post-counterstain: 8 minutes at 37°C with Hematoxylin II (VMSI Catalog #: 790-2208), rinse, Post-counterstain 4 minutes at 37°C with Bluing Reagent (VMSI Catalog #: 760-2037).

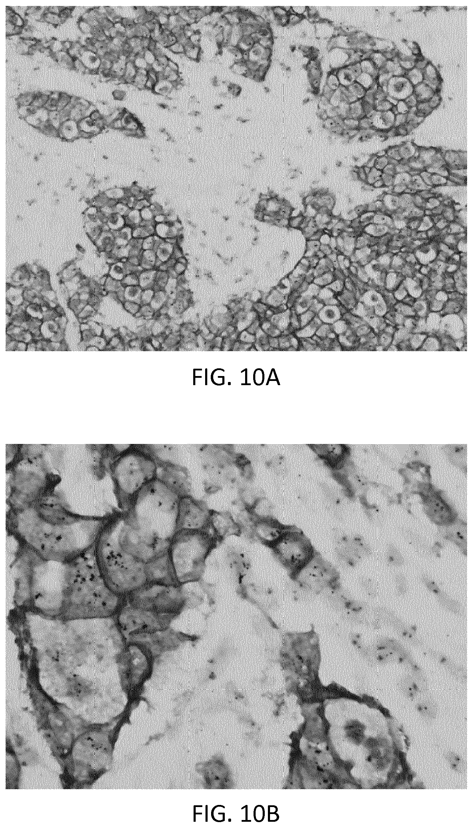

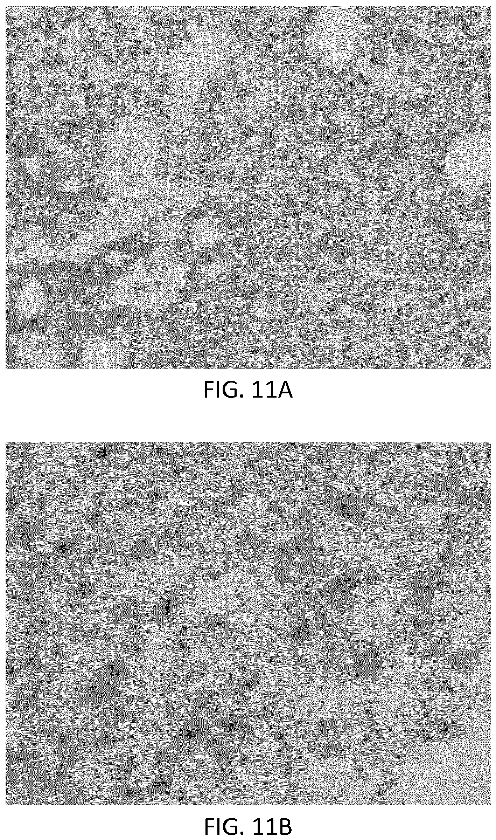

The staining protocol results in brown staining of HER2 protein, purple staining of ER protein, black staining of the HER2 genomic DNA, and red staining of chromosome 17 centromere DNA. A representative sample which has amplified HER2 gene, is HER2 protein positive, and ER protein positive is shown in FIGS. 10A and B. A sample which is considered HER2 negative (protein and gene) and ER positive is shown in FIGS. 11A and B.

### Example 4

### Fourplex HER2 and ER Gene-Protein Assay

This example describes a multiplex gene-protein assay for detection of HER2 protein, ER protein, HER2 gene copy number, and chromosome 17 copy number in a sample using single strand oligonucleotide probes for HER2 and chromosome 17 copy number analysis. See U.S. Application Ser. No. 61/943,196 for disclosure related to oligonucleotide probes. The use of the single strand oligonucleotide probes decreases the time required for the assay as the probes hybridize much more quickly than the aforementioned DNA probes (HER2 DNP and Chr17 DIG probe cocktail (VMSI catalog #780-4422). In particular, the hybridization time was decreased from 6 hours to 1 hour. Furthermore, it was discovered that HybClear solution (VMSI catalog #780-4572) was not needed for the single strand oligonucleotide probes.

The single strand oligonucleotide HER2 probe (HER2 oligonucleotide probe) is a dinitrophenyl (DNP)-labeled, repeat-free genomic probe specifically targeting the HER2 gene region. Similar to INFORM HER2 DUAL ISH DNA Probe, the HER2 oligonucleotide probe spans > 327,000 nucleotides (nt) (35,027,979 - 35,355,516) of genomic DNA from human Chromosome 17, encompassing the HER2 target region (UCSC Genome Browser on Human May 2004 (NCBI35/hg17) Assembly). The HER2 oligonucleotide sequences were designed from the sequences in INFORM HER2 DUAL ISH DNA Probe. Each of the HER2 oligonucleotides was designed with 80-mer length; hence stringency level for non-target binding was raised higher according to the aforementioned oligonucleotide probe design criteria. Specificity of the HER2 oligonucleotide probe was experimentally validated on metaphase spreads under the examined ISH assay conditions.

Bioinformatic searches were used to identify HER2 specific nucleic acid sequences around the HER2 target region. The selected genomic target nucleic acid sequence is separated into consecutive non-overlapping 80 nt segments. One thousand one hundred and ninety-six (1196) ∼80mer oligonucleotides were synthesized each carrying 5 DNP haptens on an abasic phosphoramidite spaced 20 nt apart. The oligonucleotides were affinity purified and analyzed by mass spectrometry and gel electrophoresis. HER2 oligonucleotide probe was bulked in a formamide-based buffer without human blocking DNA. In the initial screening process, the number of oligonucleotides, the number and spacing of DNP haptens were functionally tested in the formamide-based buffer without human blocking DNA for sensitivity and specificity to HER2 gene.

A single strand oligonucleotide Chr17 probe (Chr17 oligonucleotide probe) was made with a pool of 14 oligonucleotides with lengths from 58bp to 87bp. Each oligonucleotide was labeled with two DIG hapten molecules on a non-binding tail having the sequence TATTTTTATTTT at its 5' end. These oligonucleotides were PAGE purified and analyzed with mass spectrometry. The Chr17 oligonucleotide probe was formulated in a formamide-based buffer without human blocking DNA. In illustrative embodiments, the Chr 17 comprises one or more of the sequences listed in Table 2.

**Table 2: Chromosome 17 sequences.**

| **Oligo name** | **Sequences** | **Length** |
|---|---|---|
| CHR17_M1.1 SEQ ID. NO. 1 | | 79 |
| CHR17_M2.1 SEQ ID. NO. 2 | | 79 |
| CHR17_M2.2 SEQ ID. NO. 3 | | 79 |
| CHR17_M3.1 SEQ ID. NO. 4 | | 79 |
| CHR17_M5.1 SEQ ID. NO. 5 | | 83 |
| CHR17_M5.2 SEQ ID. NO. 6 | | 87 |
| CHR17_M8.2 SEQ ID. NO. 7 | | 71 |
| CHR17_M9.1 SEQ ID. NO. 8 | | 58 |
| CHR17_M9.2 SEQ ID. NO. 9 | | 65 |
| CHR17_ M11.2 SEQ ID. NO. 10 | | 71 |
| CHR17_M12.1 SEQ ID. NO. 11 | | 80 |
| CHR17_M13.1 SEQ ID. NO. 12 | | 80 |
| CHR17_M16.1 SEQ ID. NO. 13 | | 80 |
| CHR17_M16.2 SEQ ID. NO. 14 | | 79 |

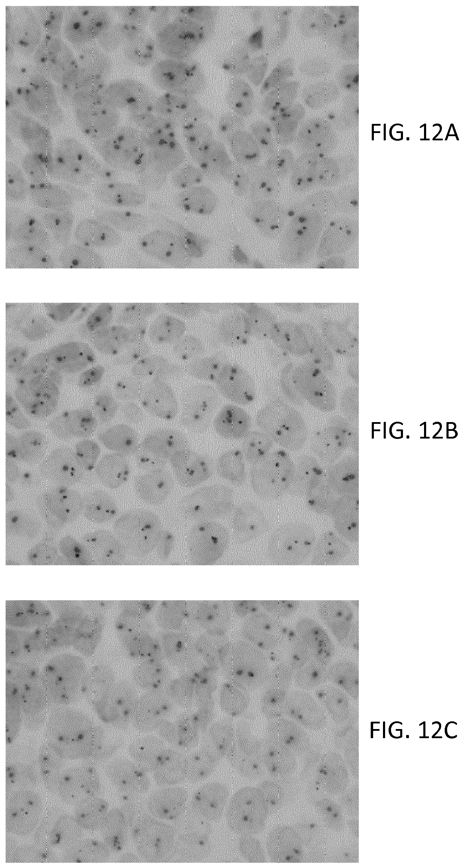

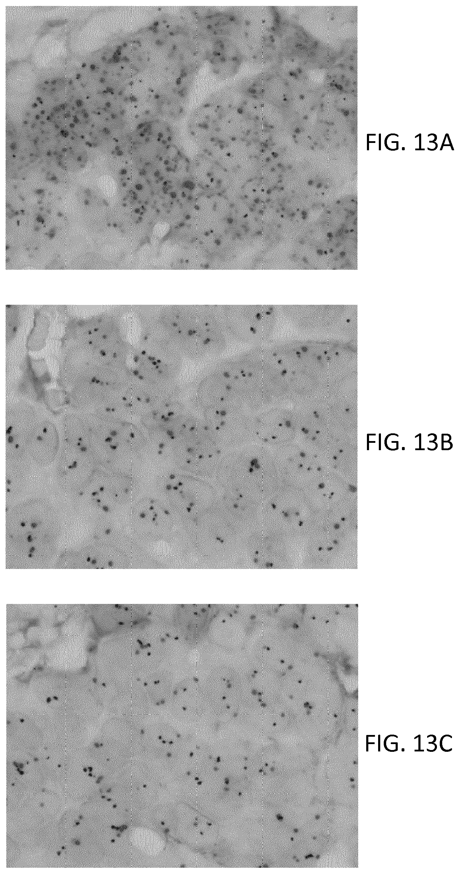

One aspect of the present invention is that in order to balance the signal between the Chr 17 and HER2 gene detections, different stringency washes are needed. In particular, it is important that when reading a gene protein assay that the gene signal (e.g. HER2) and the centromere signal (e.g. Chr 17) be of equivalent size, with both having discrete, round, readily discernible signals. Large, misshapen, disparate, or weak signals confound the reading of the gene protein assay. This issue is further confounded by higher levels of multiplexing (i.e. a four-plex or above). Accordingly, FIG. 12A-C show three photomicrographs of a cervical dysplasia case in which 12A uses a stringency wash of 68°C, 12B uses a stringency wash of 72°C, and 12C uses a stringency wash of 76°C. These tests at varied stringencies showed that a stringency wash of 68°C produced the best signal for HER2 (detection in black, SISH). FIG. 13A-C show three photomicrographs of a ZR-75-1 xenograft tumor in which 13A uses a stringency wash of 68°C, 13B uses a stringency wash of 72°C, and 13C uses a stringency wash of 76°C. It was determined that a stringency wash of 76°C produced the best signal for Chr17 (detection in red). As such, it was discovered that the four-plex gene protein assay was clearest when the HER2 was washed for stringency at 68°C, the HER2 was detected, and the Chr17 was washed for stringency at 76°C and then detected ("dual stringency wash approach").

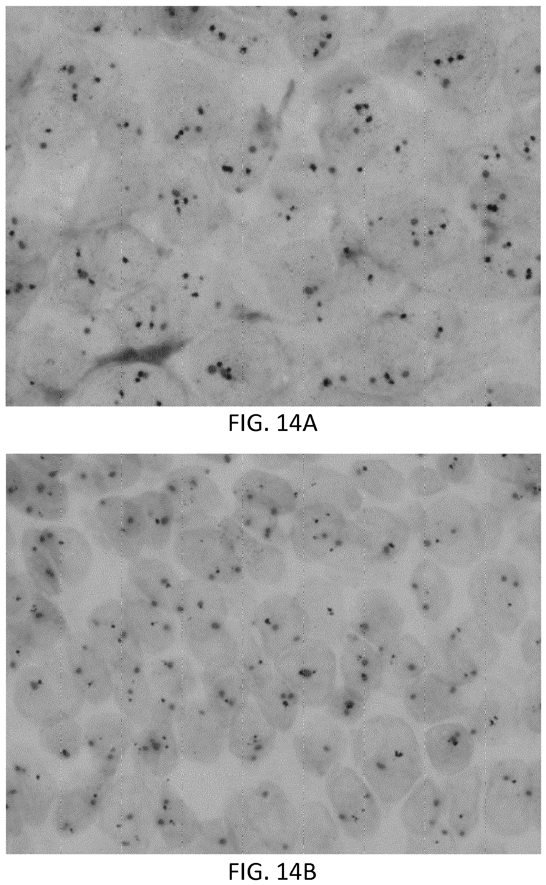

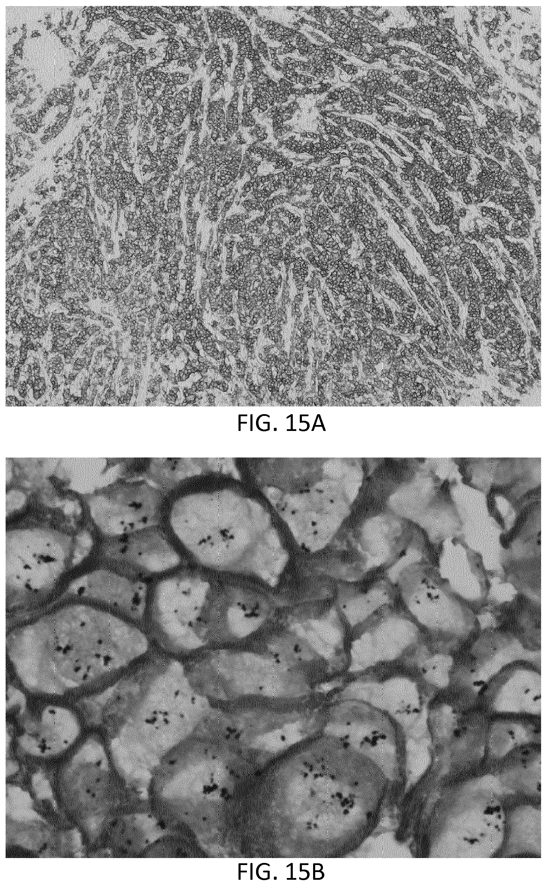

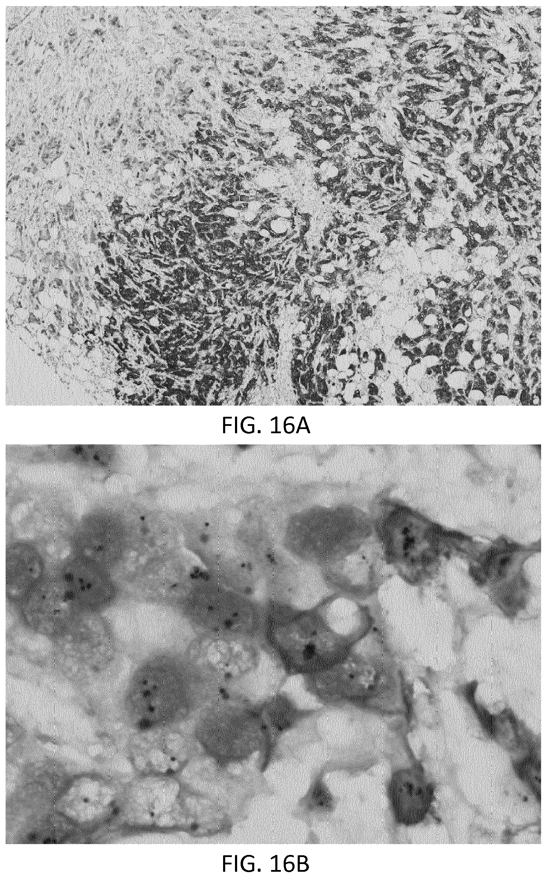

Referring now to FIG. 14A-B, shown are photomicrographs of the HER2 Gene-Protein Assay employing this dual stringency wash approach in which FIG. 14A shows a ZR-75-1 xenograft tumor and FIG. 14B shows a cervical dysplasia case. Similarly, FIGS. 15A-B show the HER2 Gene-Protein Assay employing this dual stringency wash approach in which FIG. 15A shows a breast cancer tumor at Objective 4X and FIG. 15B shows the same case at Objective 100X. In FIG. 15B, the HER2 protein is detected with DAB (brown), the ER is detected with Red, the HER2 gene is detected with SISH (black), and the Chr17 is detected with blue. Similarly, FIGS. 16A-B show the HER2 Gene-Protein Assay employing this dual stringency wash approach in which FIG. 16A shows a breast cancer tumor at Objective 4X and FIG. 16B shows the same case at Objective 100X. The various markers are detected as described for FIG 15. Of particular significance, FIG. 16A shows significant tumor heterogeneity with respect to HER2 expression. In particular, the left half of the field of view exhibits low HER2 expression whereas the right half is strongly HER2 expressing. FIG. 16B shows a 100X view of the interface between these heterogeneous portions of the tumor so it is possible to see several cells with high HER2 expression on the right and low expression on the left. Of particular interest is the observation that those cells on the left of FIG. 16B exhibit HER2 gene amplification, but not amplified HER2 protein expression. One aspect of the present disclosure is that the ability to read multi-plexed HER2 protein, ER protein, HER2 gene, Chr17 gene, enables an understanding of the heterogeneity of a tumor heretofore not possible. As such, the presently described assay provides the pathologist with an incredibly valuable tool for diagnosis.

### Example 5

### HETEROGENEITY STUDY

### Background:

The eligibility of HER2-targeted therapies for breast cancer patients is determined by the evaluation of HER2 gene amplification and HER2 protein overexpression. The gene-protein assay (GPA, Ventana Medical Systems, Inc., USA) is a new method for simultaneous evaluation of HER2 immunohistochemistry (IHC) and dual in situ hybridization (DISH) using a single tissue section. In this study, we investigated the relationship between HER2 IHC and DISH results evaluated by GPA. In addition, we analyzed the correlation between HER2 status and prognosis of invasive breast cancer patients.

### Patients and Methods:

In this study, invasive carcinoma tissues of consecutive 280 patients treated in Saitama Cancer Center in 2000-2001 (median follow-up: 130 months) were examined. In HER2 positive patients, no patients received adjuvant trastuzumab therapy. However, 76% of HER2 positive recurrent patients received trastuzumab therapy after the recurrence. GPA was performed on a section of routinely processed primary tumors and the status of HER gene and protein were separately evaluated in whole area of tumor sections using FDA criteria as followings; DISH (negative: HER2/CEN17 < 2, positive: HER2/CEN17 ≥ 2.0) and IHC (score 0 to 3+). In IHC score 2+ patients group, final HER2 positivity was decided according to DISH results using criteria of ASCO/CAP 2013 guideline. Recurrence-free survival (RFS) and cancer-specific survival (CSS) stratified by IHC and DISH results were analyzed. In addition, patterns of heterogeneity were categorized by co-presence of the following 4 phenotypic and genotypic types: A) IHC 2+/DISH+; B) IHC 2+/DISH-; C) IHC 1+ or 0/DISH+; and D) IHC 1+ & 0/DISH-. Presence of heterogeneity and prognosis was analyzed in IHC 0 & 1+ group.

### Results:

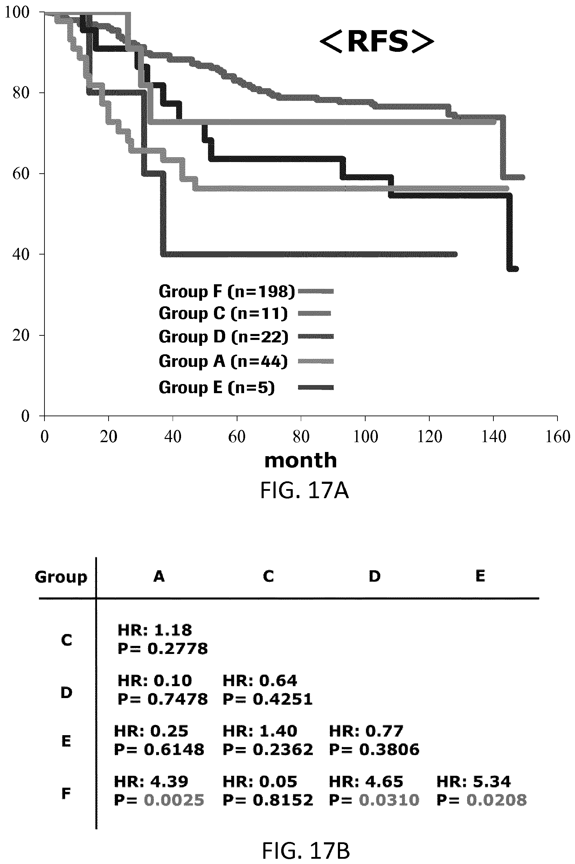

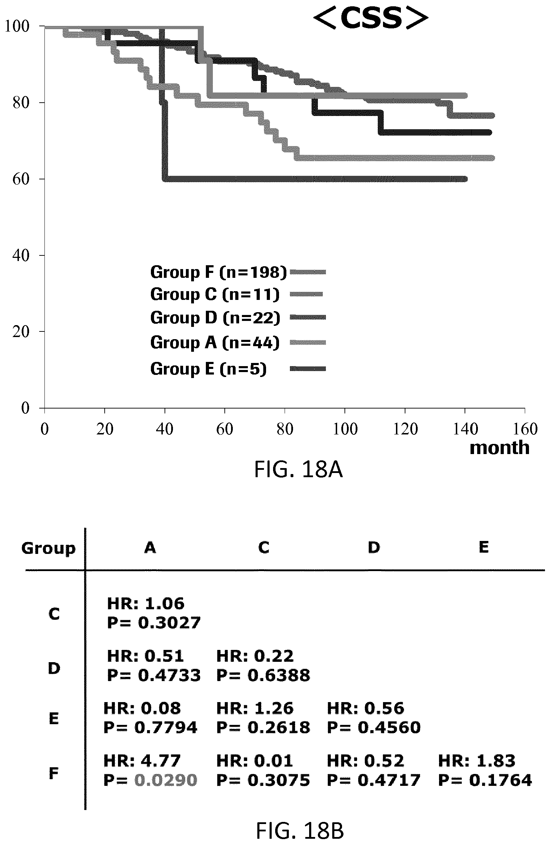

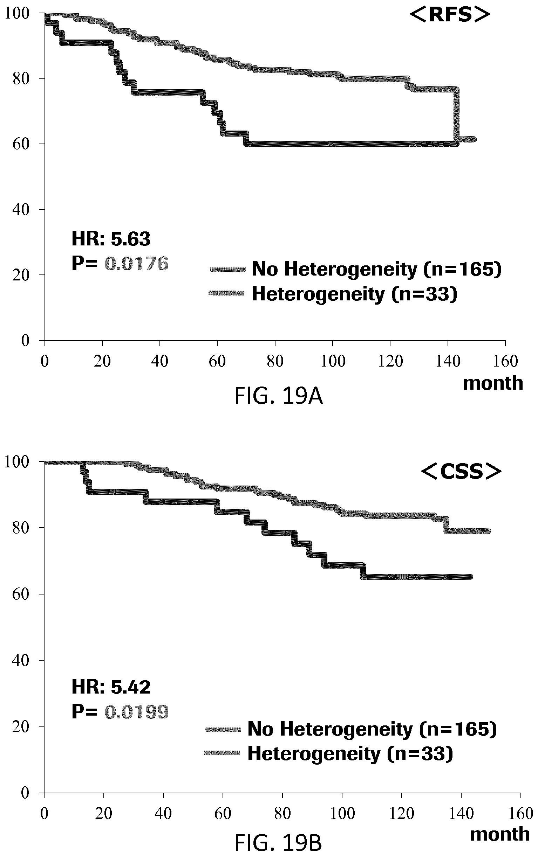

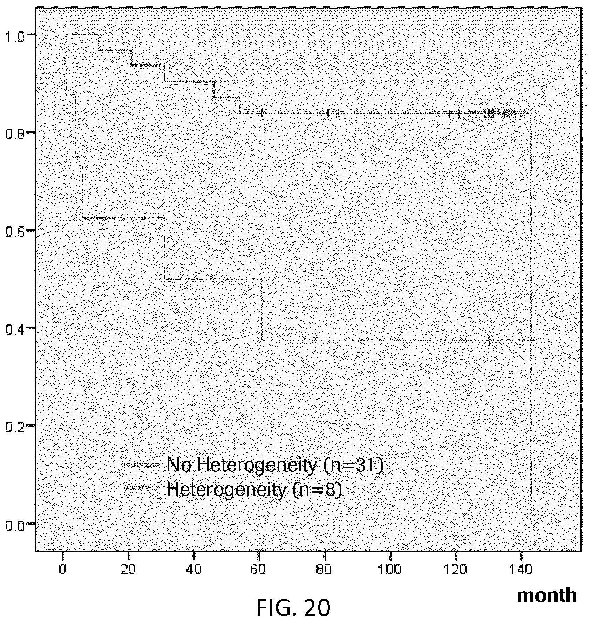

HER2 IHC 3+ group (27.5%) had significantly worse survival than HER2 IHC 1+ & 0 group (RFS: *P*=0.0039; CSS: *P*=0.0362) and HER2 DISH+ group (27.5%) had significantly worse survival than HER2 DISH- group (RFS: *P*=0.0056; CSS: *P*=0.0497). HER2 positive group defined by ASCO/CAP criteria had significantly worse RFS than HER2 negative group (*P*=0.0211). HER2 IHC 1+ & 0/DISH+ group had significantly worse RFS than IHC 1+ & 0/DISH- group (*P*=0.0208). In the HER2 IHC 1+ & 0/ DISH- group, patients with heterogeneity (33 cases) had significantly worse survival than those without heterogeneity (RFS: *P*=0.0176; CSS: *P*=0.0199). Referring now to FIGS. 17A-B, the graph (17A) and table (17B) show regression free survival (RFS) by group as determined by the gene-protein assay. Referring now to FIG. 18A and 18B, the graph and table show cancer-specific survival (CSS) by group as determined by the gene-protein assay. Referring now to FIG. 19A and 19B, shown is the utility of evaluating heterogeneity within the context of the gene protein assay. FIG. 20 shows a sub-population of the data shown in FIG. 19, wherein the population was triple negative breast cancer (TNBC - for ER, PR, and within Group F for gene protein assay). Heterogeneity in this group (n=31, non-heterogeneous; n=8, heterogeneous) was more significant (p=0.016 HR: 5.85).

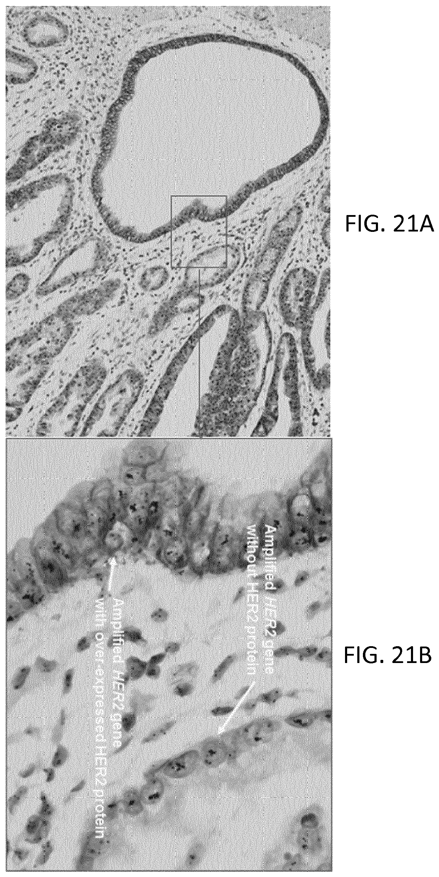

HER2 GPA technology might be useful for evaluating the discrepancy and heterogeneity of HER2 IHC and DISH results at single cell levels simultaneously and the presence of HER2 tumor cell heterogeneity might be a potent prognostic factor in HER2 negative breast cancer patients. Further clinical research must be conducted for concerning the relationship between the presence of HER2 intratumoral heterogeneity and the effectiveness of HER2-targeted therapies. Referring now to FIG. 21A-B, shown is a representative tissue stained with both HER2 gene and HER2 protein (FIG. 21A shown with a 10x objective and FIG. 21B, a 60x objective). Considering this particular breast cancer case, it may explain why a Group F scoring with heterogeneity had a poor prognosis based on the clinical study results. While some tumor cells had HER2 gene and protein positivity, other regions showed HER2 gene amplification without HER2 protein expression. The heterogeneity is mainly due to Group D (HER2 IHC negative & DISH positive) cell in Group F tumor cases (HER2 IHC negative & DISH negative). It is hypothesized that HER2 gene amplification occurs first and HER2 protein positivity may only be observed later. In FIG. 21A, the image shows multiple layers of HER2 IHC & DISH positive tumor cells and a single cell layer of HER2 IHC negative & DISH positive cells. The current breast HER2 gene and protein assay suggests that both HER2 IHC and ISH assays would enhance diagnostic capability for prognosing and predicting outcomes for breast cancer cases.

In view of the many possible embodiments to which the principles of the disclosure may be applied, it should be recognized that the illustrated embodiments are only examples and should not be taken as limiting the scope of the invention. Rather, the scope of the invention is defined by the following claims. We therefore claim as our invention all that comes within the scope of these claims.

## Claims

1. An in vitro method for predicting responsiveness to a HER2-directed therapy by assessing HER2 heterogeneity in a tumor, the method comprising:
• contacting a sample of the tumor with an antibody that specifically binds to HER2 protein and detecting HER2 protein in the sample,
• contacting the sample of the tumor with a nucleic acid probe that specifically binds HER2 genomic DNA and detecting HER2 gene amplification status in the sample,
• scoring the HER2 protein (IHC) and HER2 gene (DISH), wherein scoring is categorized as:
∘ Group A for samples exhibiting IHC 3+ and DISH+,
∘ Group B for samples exhibiting IHC 3+ and DISH-,
∘ Group C for samples exhibiting IHC 2+ and DISH+,
∘ Group D for samples exhibiting IHC 2+ and DISH-,
∘ Group E for samples exhibiting IHC 0, 1+ and DISH+, and
∘ Group F for samples exhibiting IHC 0, 1+ and DISH-,
• predicting that the tumor is responsive to the HER2-directed therapy if the tumor reveals a first foci having a first score being Group F and a second foci having a second score selected from Group A to Group E.

2. The method of claim 1, wherein the method further comprises assaying a second sample of the tumor for estrogen receptor (ER) and progesterone receptor (PR), wherein the tumor is predicted as being responsive to the HER2-directed therapy if the ER and PR are negative so that the tumor is understood to be triple negative breast cancer (TNBC).

3. The method of claim 1, wherein the method further comprises
• contacting the sample of the tumor with an antibody that specifically binds to estrogen receptor (ER) protein and detecting ER protein in the sample;
• contacting the sample of the tumor with an antibody that specifically binds to progesterone receptor (PR) protein and detecting PR protein in the sample,
wherein the tumor is predicted as being responsive to the HER2-directed therapy if the ER and PR are negative so that the tumor is understood to be triple negative breast cancer (TNBC).

4. The method of any of claims 1 to 3, wherein the HER-2 directed therapy is selected from the group consisting of trastuzumab, trastuzumab emtansine, pertuzumab, neratinib, and lapatinib.

5. An in vitro method of scoring a tumor sample, the method comprising:
• contacting the tumor sample with an antibody that specifically binds to HER2 protein and detecting HER2 protein in the sample,
• contacting the tumor sample with a nucleic acid probe that specifically binds HER2 genomic DNA and detecting HER2 gene amplification status in the sample,
• scoring the HER2 protein (IHC) and HER2 gene (DISH), wherein scoring is categorized as:
∘ Group A for samples exhibiting IHC 3+ and DISH+,
∘ Group B for samples exhibiting IHC 3+ and DISH-,
∘ Group C for samples exhibiting IHC 2+ and DISH+,
∘ Group D for samples exhibiting IHC 2+ and DISH-,
∘ Group E for samples exhibiting IHC 0, 1+ and DISH+, and
∘ Group F for samples exhibiting IHC 0, 1+ and DISH-,
• scoring the tumor sample as heterogeneous if the tumor reveals a first foci having a first score being Group F and a second foci having a second score selected from Group A to Group E.

6. The method of claim 5, wherein the method further comprises prognosing a hazard ratio of greater than 5 if the tumor sample is scored as heterogeneous.

7. The method of claim 5, wherein the method further comprises assaying a second sample of the tumor for estrogen receptor (ER) and progesterone receptor (PR), wherein the tumor is predicted as being responsive to a HER2-directed therapy if the ER and PR are negative so that the tumor is understood to be triple negative breast cancer (TNBC).

8. The method of claim 5, wherein the method further comprises
• contacting the sample of the tumor with an antibody that specifically binds to estrogen receptor (ER) protein and detecting ER protein in the sample;
• contacting the sample of the tumor with an antibody that specifically binds to progesterone receptor (PR) protein and detecting PR protein in the sample,
wherein the tumor is predicted as being responsive to a HER2-directed therapy if the ER and PR are negative so that the tumor is understood to be triple negative breast cancer (TNBC).

9. The method of claim 7 or 8, wherein the method further comprises prognosing a significantly worse survival score compared to a non-heterogeneous score (RFS: P=0.0176; CSS: P=0.0199) if the sample is scored as heterogeneous.

## Patentansprüche

1. *In-vitro*-Verfahren zum Prognostizieren des Ansprechens auf eine gegen HER2 gerichtete Therapie durch die Beurteilung der HER2-Heterogenität in einem Tumor, wobei das Verfahren Folgendes umfasst:
• Inkontaktbringen einer Probe des Tumors mit einem Antikörper, der spezifisch an HER2-Protein bindet, und Detektieren von HER2-Protein in der Probe,
• Inkontaktbringen einer Probe des Tumors mit einer Nukleinsäuresonde, die spezifisch an genomische HER2-DNS bindet, und Detektieren des HER2-Genamplifikationsstatus in der Probe,
• Bewerten des HER2-Proteins (IHC) und HER2-Gens (DISH), wobei es folgende Bewertungskategorien gibt:
∘ Gruppe A für Proben, die IHC 3+ und DISH+ aufweisen,
∘ Gruppe B für Proben, die IHC 3+ und DISH- aufweisen,
∘ Gruppe C für Proben, die IHC 2+ und DISH+ aufweisen,
∘ Gruppe D für Proben, die IHC 2+ und DISH- aufweisen,
∘ Gruppe E für Proben, die IHC 0, 1+ und DISH+ aufweisen, und
∘ Gruppe F für Proben, die IHC 0, 1+ und DISH- aufweisen,
• Prognostizieren, dass der Tumor auf die gegen HER2 gerichtete Therapie anspricht, wenn der Tumor einen ersten Herd mit einem ersten Wert entsprechend der Gruppe F und einen zweiten Herd mit einem zweiten Wert ausgewählt aus der Gruppe A bis Gruppe E aufweist.

2. Verfahren nach Anspruch 1, wobei das Verfahren ferner das Untersuchen einer zweiten Probe des Tumors auf Östrogenrezeptor (ER) und Progesteronrezeptor (PR) umfasst, wobei prognostiziert wird, dass der Tumor auf die gegen HER2 gerichtete Therapie anspricht, wenn der ER und der PR negativ sind, so dass der Tumor als dreifach negativer Brustkrebs (TNBC) verstanden wird.

3. Verfahren nach Anspruch 1, wobei das Verfahren ferner Folgendes umfasst
• Inkontaktbringen der Probe des Tumors mit einem Antikörper, der spezifisch an Östrogenrezeptor(ER)-Protein bindet, und Detektieren von ER-Protein in der Probe;
• Inkontaktbringen der Probe des Tumors mit einem Antikörper, der spezifisch an Progesteronrezeptor(PR)-Protein bindet, und Detektieren von PR-Protein in der Probe,
wobei prognostiziert wird, dass der Tumor auf die gegen HER2 gerichtete Therapie anspricht, wenn der ER und der PR negativ sind, so dass der Tumor als dreifach negativer Brustkrebs (TNBC) verstanden wird.

4. Verfahren nach einem der Ansprüche 1 bis 3, wobei die gegen HER-2 gerichtete Therapie ausgewählt ist aus der Gruppe bestehend aus Trastuzumab, Trastuzumab Emtansin, Pertuzumab, Neratinib und Lapatinib.

5. *In-vitro*-Verfahren zum Bewerten einer Tumorprobe, wobei das Verfahren Folgendes umfasst:
• Inkontaktbringen der Tumorprobe mit einem Antikörper, der spezifisch an HER2-Protein bindet, und Detektieren von HER2-Protein in der Probe,
• Inkontaktbringen der Tumorprobe mit einer Nukleinsäuresonde, die spezifisch an genomische HER2-DNS bindet, und Detektieren des HER2-Genamplifikationsstatus in der Probe,
• Bewerten des HER2-Proteins (IHC) und HER2-Gens (DISH), wobei es folgende Bewertungskategorien gibt:
∘ Gruppe A für Proben, die IHC 3+ und DISH+ aufweisen,
∘ Gruppe B für Proben, die IHC 3+ und DISH- aufweisen,
∘ Gruppe C für Proben, die IHC 2+ und DISH+ aufweisen,
∘ Gruppe D für Proben, die IHC 2+ und DISH- aufweisen,
∘ Gruppe E für Proben, die IHC 0, 1+ und DISH+ aufweisen, und
∘ Gruppe F für Proben, die IHC 0, 1+ und DISH- aufweisen,
• Bewerten der Tumorprobe als heterogen, wenn der Tumor einen ersten Herd mit einem ersten Wert entsprechend der Gruppe F und einen zweiten Herd mit einem zweiten Wert ausgewählt aus der Gruppe A bis Gruppe E aufweist.

6. Verfahren nach Anspruch 5, wobei das Verfahren ferner das Prognostizieren eines Risikoquotienten (Hazard Ratio) von mehr als 5, wenn die Tumorprobe als heterogen bewertet ist, umfasst.

7. Verfahren nach Anspruch 5, wobei das Verfahren ferner das Untersuchen einer zweiten Probe des Tumors auf Östrogenrezeptor (ER) und Progesteronrezeptor (PR) umfasst, wobei prognostiziert wird, dass der Tumor auf eine gegen HER2 gerichtete Therapie anspricht, wenn der ER und der PR negativ sind, so dass der Tumor als dreifach negativer Brustkrebs (TNBC) verstanden wird.

8. Verfahren nach Anspruch 5, wobei das Verfahren ferner Folgendes umfasst
• Inkontaktbringen der Probe des Tumors mit einem Antikörper, der spezifisch an Östrogenrezeptor(ER)-Protein bindet, und Detektieren von ER-Protein in der Probe;
• Inkontaktbringen der Probe des Tumors mit einem Antikörper, der spezifisch an Progesteronrezeptor(PR)-Protein bindet, und Detektieren von PR-Protein in der Probe,
wobei prognostiziert wird, dass der Tumor auf eine gegen HER2 gerichtete Therapie anspricht, wenn der ER und der PR negativ sind, so dass der Tumor als dreifach negativer Brustkrebs (TNBC) verstanden wird.

9. Verfahren nach Anspruch 7 oder 8, wobei das Verfahren ferner das Prognostizieren eines signifikant schlechteren Überlebenswerts im Vergleich zu einem nicht heterogenen Wert (RFS: P=0,0176; CSS: P=0,0199), wenn die Probe als heterogen bewertet ist, umfasst.

## Revendications

1. Une méthode in vitro pour prédire une réceptivité à une thérapie dirigée contre HER2 en évaluant l'hétérogénéité d'HER2 dans une tumeur, la méthode comprenant :
• la mise en contact d'un échantillon de la tumeur avec un anticorps qui se lie spécifiquement à la protéine HER2 et la détection de la protéine HER2 dans l'échantillon,
• la mise en contact de l'échantillon de la tumeur avec une sonde d'acide nucléique qui se lie spécifiquement à l'ADN génomique d'HER2 et la détection d'un statut d'amplification du gène HER2 dans l'échantillon,
• l'évaluation par score de la protéine HER2 (IHC) et du gène HER2 (DISH), dans laquelle l'évaluation par score est catégorisée comme :
∘ Groupe A pour des échantillons présentant IHC 3+ et DISH+,
∘ Groupe B pour des échantillons présentant IHC 3+ et DISH-,
∘ Groupe C pour des échantillons présentant IHC 2+ et DISH+,
∘ groupe D pour des échantillons présentant IHC 2+ et DISH-,
∘ Groupe E pour des échantillons présentant IHC 0, 1+ et DISH+, et
∘ Groupe F pour des échantillons présentant IHC 0, 1+ et DISH-,
• la prédiction que la tumeur est réceptive à une thérapie dirigée contre HER2 si la tumeur révèle un premier foyer ayant un premier score qui est le groupe F et un second foyer ayant un second score sélectionné du groupe A au groupe E.

2. La méthode selon la revendication 1, dans laquelle la méthode comprend en outre l'analyse d'un second échantillon de la tumeur pour un récepteur des oestrogènes (ER) et un récepteur de la progestérone (PR), dans laquelle la tumeur est supposée réceptive à la thérapie dirigée contre HER2 si les ER et PR sont négatifs de telle sorte que la tumeur est considérée comme un cancer du sein triple négatif (TNBC).

3. La méthode selon la revendication 1, dans laquelle la méthode comprend en outre
• la mise en contact de l'échantillon de la tumeur avec un anticorps qui se lie spécifiquement à une protéine d'un récepteur des oestrogènes (ER) et la détection d'une protéine ER dans l'échantillon ;
• la mise en contact de l'échantillon de la tumeur avec un anticorps qui se lie spécifiquement à une protéine d'un récepteur de la progestérone (PR) et la détection d'une protéine PR dans l'échantillon ;
dans laquelle la tumeur est supposée être réceptive à la thérapie dirigée contre HER2 si les ER et PR sont négatifs de telle sorte que la tumeur est considérée comme un cancer du sein triple négatif (TNBC).

4. La méthode selon l'une quelconque des revendications 1 à 3, dans laquelle la thérapie dirigée contre HER2 est sélectionnée dans le groupe constitué par le trastuzumab, le trastuzumab emtansine, le pertuzumab, le nératinib et le lapatinib.

5. Une méthode in vitro d'évaluation par score d'un échantillon tumoral, la méthode comprenant :
• la mise en contact de l'échantillon tumoral avec un anticorps qui se lie spécifiquement à la protéine HER2 et la détection de la protéine HER2 dans l'échantillon,
• la mise en contact de l'échantillon tumoral avec une sonde d'acide nucléique qui se lie spécifiquement à l'ADN génomique d'HER2 et la détection d'un statut d'amplification du gène HER2 dans l'échantillon,
• l'évaluation par score de la protéine HER2 (IHC) et du gène HER2 (DISH), dans laquelle l'évaluation par score est catégorisée comme :
∘ Groupe A pour des échantillons présentant IHC 3+ et DISH+,
∘ Groupe B pour des échantillons présentant IHC 3+ et DISH-,
∘ Groupe C pour des échantillons présentant IHC 2+ et DISH+,
∘ groupe D pour des échantillons présentant IHC 2+ et DISH-,
∘ Groupe E pour des échantillons présentant IHC 0, 1+ et DISH+, et
∘ Groupe F pour des échantillons présentant IHC 0, 1+ et DISH-,
• l'évaluation par score de l'échantillon tumoral comme hétérogène si la tumeur révèle un premier foyer ayant un premier score qui est le groupe F, et un second foyer ayant un second score sélectionné du groupe A au groupe E.

6. La méthode selon la revendication 5, dans laquelle la méthode comprend en outre le pronostic d'un rapport de risque supérieur à 5 si l'échantillon tumoral est évalué par score comme hétérogène.

7. La méthode selon la revendication 5, dans laquelle la méthode comprend en outre l'analyse d'un second échantillon de la tumeur pour un récepteur des oestrogènes (ER) et un récepteur de la progestérone (PR), dans laquelle la tumeur est supposée réceptive à une thérapie dirigée contre HER2 si les ER et PR sont négatifs de telle sorte que la tumeur est considérée comme un cancer du sein triple négatif (TNBC).

8. La méthode selon la revendication 5, dans laquelle la méthode comprend en outre :
• la mise en contact de l'échantillon de la tumeur avec un anticorps qui se lie spécifiquement à une protéine d'un récepteur des oestrogènes (ER) et la détection d'une protéine ER dans l'échantillon ;
• la mise en contact de l'échantillon de la tumeur avec un anticorps qui se lie spécifiquement à une protéine d'un récepteur de la progestérone (PR) et la détection d'une protéine PR dans l'échantillon ;
dans laquelle la tumeur est supposée être réceptive à une thérapie dirigée contre HER2 si les ER et PR sont négatifs de telle sorte que la tumeur est considérée comme un cancer du sein triple négatif (TNBC).

9. La méthode selon la revendication 7 ou 8, dans laquelle la méthode comprend en outre le pronostic d'un score de survie significativement pire par comparaison avec un score non hétérogène (RFS : P = 0,0176 ; CSS : P = 0,0199) si l'échantillon est évalué par score comme hétérogène.


### SEQUENCE LISTING

<110> Ventana Medical Systems, Inc.
<120> SIGNIFICANCE OF INTRATUMORAL HER2 HETEROGENIETY IN BREAST CANCER AND USES THEREFORE
<130> 32156
<150> US62/009057
   <151> 2014-06-06
<160> 14
<170> PatentIn version 3.5
<210> 1
   <211> 79
   <212> DNA
   <213> Homo sapiens
<400> 1
   aattcgttgg aaacgggata atttcagctg actaaacaga agcagtctca gaatcttctt        60
   tgtgatgttt gcattcaaa                                                     79
<210> 2
   <211> 79
   <212> DNA
   <213> Homo sapiens
<400> 2
   cttcgttcga aacgggtata tcttcacatg ccatctagac agaagcatcc tcagaagctt        60
   ctctgtgatg actgcattc                                                     79
<210> 3
   <211> 79
   <212> DNA
   <213> Homo sapiens
<400> 3
   tgaactctcc ttttgagagc gcagttttga aactctcttt ctgtggcatc tgcaagggga        60
   catgtagacc tctttgaag                                                     79
<210> 4
   <211> 79
   <212> DNA
   <213> Homo sapiens
<400> 4
   tttcgttgga aacggaatca tcttcacata aaaactacac agatgcattc tcaggaactt        60
   tttggtgatg tttgtattc                                                     79
<210> 5
   <211> 83
   <212> DNA
   <213> Homo sapiens
<400> 5
   cctatggtag taaagggaat agcttcatag aaaaactaga cagaagcatt ctcagaaaat 60
   actttgtgat gattgagttt aac 83
<210> 6
   <211> 87
   <212> DNA
   <213> Homo sapiens
<400> 6
   cacagagctg aacattcctt tggatggagc aggtttgaga cactcttttt gtacaatcta 60
   caagtggata tttggacctc tctgagg 87
<210> 7
   <211> 71
   <212> DNA
   <213> Homo sapiens
<400> 7
   gtttcacatt gcttttcata gagtagttct gaaacatgct tttcgtagtg tctacaagtg 60
   gacatttgga g 71
<210> 8
   <211> 58
   <212> DNA
   <213> Homo sapiens
<400> 8
   cctgtggtgg aaaacgaatt atcgtcacgt aaaaactaga gagaagcatt gtcagaaa 58
<210> 9
   <211> 65
   <212> DNA
   <213> Homo sapiens
<400> 9
   tgcattcaac tcacagagtt gaaggttcct tttcaaagag cagtttccaa tcactctttg 60
   tgtgg 65
<210> 10
   <211> 71
   <212> DNA
   <213> Homo sapiens
<400> 10
   cattcccttt gacagagcag tttggaaact ctctttgtgt agaatctgca agtggagata 60
   tggaccgctt t 71
<210> 11
   <211> 80
   <212> DNA
   <213> Homo sapiens
<400> 11
   cctatggtag taaaggaaat agcttcatat aaaagctaga cagtagcatt cacagaaaac 60
   tcttggtgac gactgagttt 80
<210> 12
   <211> 80
   <212> DNA
   <213> Homo sapiens
<400> 12
   atttcgttgg aaacgggata aaccgcacag aactaaacag aagcattctc agaaccttct 60
   tcgtgatgtt tgcattcaac 80
<210> 13
   <211> 80
   <212> DNA
   <213> Homo sapiens
<400> 13
   cgtagtaaag gaaataactt cctataaaaa gaagacagaa gctttctcag aaaattcttt 60
   gggatgattg agttgaactc 80
<210> 14
   <211> 79
   <212> DNA
   <213> Homo sapiens
<400> 14
   acagagctga gcattccttg cgatgtagca gtttagaaac acactttctg cagaatctgc 60
   aattgcatat ttggacctt 79